# EUROPEAN PATENT APPLICATION

(11) **EP 4 711 384 A1**
(43) Date of publication of application: **18.03.2026**
(21) Application number: 24795455.5
(22) Date of filing: 16.01.2024
(51) Int. Cl.: C07K 16/28, C12N 15/13, C12N 15/85, C12N 5/10, A61K 39/395, A61P 35/00

(54) **ANTIBODY BINDING TO IL-18 RECEPTOR, IL-18 RECEPTOR ACTIVATOR, AND USE THEREOF**

(30) Priority: 26.04.2023 CN 202310462104
(71) Applicant: WIO Bioscience Co., Ltd., Beijing 102206 (CN)
(72) Inventor: ZENG, Dadi, Beijing 102206 (CN)
(74) Representative: Geskes, Christoph
(86) International application number: PCT/CN2024/072544
(87) International publication number: WO 2024/222066

(57) **Abstract**

The present invention relates to the technical field of antibodies, and in particular to an antibody binding to IL-18 receptor, an IL-18 receptor activator, and the use thereof. The present invention developed antibodies that specifically bind to IL-18R1 and IL-18RAP with high affinity, and also provides a bispecific antibody capable of simultaneously and efficiently binding to IL-18R1 and IL-18RAP. The bispecific antibody is a brand-new form of IL-18 receptor activator, can activate IL-18R1/IL-18RAP-mediated intracellular signal pathway, can exert biological functions similar to those of IL-18 in vitro and in vivo, has a relatively broad-spectrum immune enhancement effect and obvious anti-tumor activity, has controllable activation activity, better molecular stability and in-vivo pharmacokinetic characteristics, is more conducive to the full play of anti-tumor drug effects in vivo, and has wide anti-tumor clinical application prospects.

## Description

### FIELD

The present application relates to the field of antibodies, particularly to an antibody that binds to an IL-18 receptor, an IL-18 receptor activator, and uses thereof.

### BACKGROUND

IL-18 belongs to the IL-1 family and is an important pro-inflammatory factor (Okamura H, and et al. Cloning of a new cytokine that induces IFN-gamma production by T cells. Nature. 1995 Nov 2; 378(6552):88-91. doi: 10.1038/378088a0. PMID: 7477296.), which is mainly secreted by macrophages, dendritic cells, and epithelial cells. IL-18 exists in the form of an inactive precursor within cells. When inflammation occurs, caspase-1 in the inflammasome cleaves the precursor peptide of IL-18, resulting in the rapid release of IL-18 in its active form. The active form of IL-18 mediates the MyD88-NF κ B signaling pathway by binding to its heterodimeric receptor (IL-18R1/Rap). One of the main functions of IL-18 is to stimulate NK cells and antigen-primed lymphocytes to produce interferon-γ (Nakanishi K, Yoshimoto T, Tsutsui H, Okamura H. Interleukin-18 regulates both Th1 and Th2 responses. Annu Rev Immunol. 2001; 19:423-74. doi: 10.1146/annurev.immunol.19.1.423. PMID: 11244043.).

Due to its immune activation function, IL-18 was once used in tumor immunotherapy, but ended in failure in phase II clinical trials (Tarhini AA, and et al. A phase 2, randomized study of SB-485232, rhIL-18, in patients with previously untreated metastatic melanoma. Cancer. 2009 Feb 15; 115(4):859-68. doi: 10.1002/cncr.24100. PMID: 19140204.). A study has found that the efficacy of IL-18 gradually weakens after multiple treatments in clinical patients, and at the same time, a large amount of IL-18 binding protein (IL-18BP) is induced and produced in the body (Robertson MJ, and et al. A dose-escalation study of recombinant human interleukin-18 using two different schedules of administration in patients with cancer. Clin Cancer Res. 2008 Jun 1; 14(11):3462-9. doi: 10.1158/1078-0432.CCR-07-4740. PMID: 18519778; PMCID: PMC8603216.). IL-18BP is a decoy receptor and a natural inhibitor of IL-18, which binds to IL-18 with ultra-high affinity (1.1 pM) and prevents IL-18 from binding to the receptor. The presence of IL-18BP can antagonize the immune activation function of IL-18, leading to treatment failure.

On June 24, 2020, Nature published a research paper by Aaron Ring's research group at Yale University (Zhou T, and et al. IL-18BP is a secreted immune checkpoint and barrier to IL-18 immunotherapy. Nature. 2020 Jul; 583(7817):609-614. doi: 10.1038/s41586-020-2422-6. Epub 2020 Jun 24. PMID: 32581358; PMCID: PMC7381364.), reporting that IL-18 was engineered into DR-18 (decoy-resistant IL-18) through directed evolution, which does not bind to the decoy receptor but retains signaling function, and DR-18 exhibits promising anti-tumor efficacy. All preclinical studies and drug production for DR-18 have been completed, and an Investigational New Drug (IND) application has been submitted. Currently, the project is undergoing Phase 1/2 clinical trials. However, although DR-18 avoids the antagonistic effect of IL-18BP and exhibits clear anti-tumor efficacy and a favorable safety profile, DR-18, as a cytokine analogue, still suffers from issues such as a short in vivo half-life, difficulty in maintaining its activation activity, poor stability, and a complex production process.

### BRIEF SUMMARY

The present application provides an antibody that binds to an IL-18 receptor, an IL-18 receptor activator, and uses thereof.

To develop an antibody that binds to an IL-18 receptor and an IL-18 receptor activator, firstly, multiple single-domain antibodies with unique sequences against IL-18R1 and IL-18RAP are obtained using the camel immune library technology in the present application. These single-domain antibodies have high affinity for IL-18R1 and IL-18RAP, and can efficiently bind to IL-18 receptors. Furthermore, a tandem bispecific combinational library is constructed using the single-domain antibodies against IL-18R1 and IL-18RAP. Multiple aIL-18R1 and aIL-18RAP tandem bispecific antibodies with IL-18-like activation activity are obtained through ELISA combined with functional activity screening. On this basis, the single-domain antibodies and bispecific antibodies are also humanized in the present application, and the humanized aIL-18R1 and aIL-18RAP bispecific antibodies still maintain good cell activation activity. The aIL-18R1 and aIL-18RAP bispecific antibodies have the biological function of stimulating PBMC cell proliferation and activating the production of IFN-γ in vitro, can significantly promote the immune reconstitution of PBMC immune cells in mice, and exert dose-dependent anti-tumor efficacy. Moreover, compared with IL-18, the bispecific antibodies have mild and long-lasting activation activity and have better safety potential.

Specifically, the present application provides the following solutions.

In a first aspect, there is provided in the present application an antibody or an antigen-binding fragment thereof that binds to an IL-18 receptor, which binds to IL-18R1 or IL-18RAP and includes a heavy chain variable region;

wherein, the complementary determining regions of the heavy chain variable region of the antibody or the antigen-binding fragment thereof that binds to IL-18R1 is any one of the following (1) - (5):
(1) the amino acid sequence of the complementary determining region CDR1 of the heavy chain variable region is set forth in SEQ ID NO. 27, or it is a sequence variant having at least 80% homology to the sequence set forth in SEQ ID NO. 27,
   the amino acid sequence of the complementary determining region CDR2 of the heavy chain variable region is set forth in SEQ ID NO. 28, or it is a sequence variant having at least 80% homology to the sequence set forth in SEQ ID NO. 28,
   the amino acid sequence of the complementary determining region CDR3 of the heavy chain variable region is set forth in SEQ ID NO. 29 or SEQ ID NO. 51, or it is a sequence variant having at least 80% homology to the sequence set forth in SEQ ID NO. 29 or SEQ ID NO. 51;
(2) the amino acid sequence of the complementary determining region CDR1 of the heavy chain variable region is set forth in SEQ ID NO. 30, or it is a sequence variant having at least 80% homology to the sequence set forth in SEQ ID NO. 30,
   the amino acid sequence of the complementary determining region CDR2 of the heavy chain variable region is set forth in SEQ ID NO. 31, or it is a sequence variant having at least 80% homology to the sequence set forth in SEQ ID NO. 31,
   the amino acid sequence of the complementary determining region CDR3 of the heavy chain variable region is set forth in SEQ ID NO. 32 or SEQ ID NO. 52, or it is a sequence variant having at least 80% homology to the sequence set forth in SEQ ID NO. 32 or SEQ ID NO. 52;
(3) the amino acid sequence of the complementary determining region CDR1 of the heavy chain variable region is set forth in SEQ ID NO. 33, or it is a sequence variant having at least 80% homology to the sequence set forth in SEQ ID NO. 33,
   the amino acid sequence of the complementary determining region CDR2 of the heavy chain variable region is set forth in SEQ ID NO. 34 or SEQ ID NO. 53, or it is a sequence variant having at least 80% homology to the sequence set forth in SEQ ID NO. 34 or SEQ ID NO. 53,
   the amino acid sequence of the complementary determining region CDR3 of the heavy chain variable region is set forth in SEQ ID NO. 35, or it is a sequence variant having at least 80% homology to the sequence set forth in SEQ ID NO. 35;
(4) the amino acid sequence of the complementary determining region CDR1 of the heavy chain variable region is set forth in SEQ ID NO. 36, or it is a sequence variant having at least 80% homology to the sequence set forth in SEQ ID NO. 36,
   the amino acid sequence of the complementary determining region CDR2 of the heavy chain variable region is set forth in SEQ ID NO. 37 or SEQ ID NO. 54, or it is a sequence variant having at least 80% homology to the sequence set forth in SEQ ID NO. 37 or SEQ ID NO. 54,
   the amino acid sequence of the complementary determining region CDR3 of the heavy chain variable region is set forth in SEQ ID NO. 38 or SEQ ID NO. 55, or it is a sequence variant having at least 80% homology to the sequence set forth in SEQ ID NO. 38 or SEQ ID NO. 55; and
(5) the amino acid sequence of the complementary determining region CDR1 of the heavy chain variable region is set forth in SEQ ID NO. 39, or it is a sequence variant having at least 80% homology to the sequence set forth in SEQ ID NO. 39,
   the amino acid sequence of the complementary determining region CDR2 of the heavy chain variable region is set forth in SEQ ID NO. 40, or it is a sequence variant having at least 80% homology to the sequence set forth in SEQ ID NO. 40,
   the amino acid sequence of the complementary determining region CDR3 of the heavy chain variable region is set forth in SEQ ID NO. 41, or it is a sequence variant having at least 80% homology to the sequence set forth in SEQ ID NO. 41.

The complementary determining regions of the heavy chain variable region of the antibody or the antigen-binding fragment thereof that binds to IL-18RAP is any one of the following (6) - (8):
(6) the amino acid sequence of the complementary determining region CDR1 of the heavy chain variable region is set forth in SEQ ID NO. 42, or it is a sequence variant having at least 80% homology to the sequence set forth in SEQ ID NO. 42,
the amino acid sequence of the complementary determining region CDR2 of the heavy chain variable region is set forth in SEQ ID NO. 43 or SEQ ID NO. 56, or it is a sequence variant having at least 80% homology to the sequence set forth in SEQ ID NO. 43 or SEQ ID NO. 56,
the amino acid sequence of the complementary determining region CDR3 of the heavy chain variable region is set forth in SEQ ID NO. 44, or it is a sequence variant having at least 80% homology to the sequence set forth in SEQ ID NO. 44;
(7) the amino acid sequence of the complementary determining region CDR1 of the heavy chain variable region is set forth in SEQ ID NO. 45, or it is a sequence variant having at least 80% homology to the sequence set forth in SEQ ID NO. 45,
the amino acid sequence of the complementary determining region CDR2 of the heavy chain variable region is set forth in SEQ ID NO. 46 or SEQ ID NO. 57, or it is a sequence variant having at least 80% homology to the sequence set forth in SEQ ID NO. 46 or SEQ ID NO. 57,
the amino acid sequence of the complementary determining region CDR3 of the heavy chain variable region is set forth in SEQ ID NO. 47, or it is a sequence variant having at least 80% homology to the sequence set forth in SEQ ID NO. 47; and
(8) the amino acid sequence of the complementary determining region CDR1 of the heavy chain variable region is set forth in SEQ ID NO. 48, or it is a sequence variant having at least 80% homology to the sequence set forth in SEQ ID NO. 48,
the amino acid sequence of the complementary determining region CDR2 of the heavy chain variable region is set forth in SEQ ID NO. 49 or SEQ ID NO. 58, or it is a sequence variant having at least 80% homology to the sequence set forth in SEQ ID NO. 49 or SEQ ID NO. 58,
the amino acid sequence of the complementary determining region CDR3 of the heavy chain variable region is set forth in SEQ ID NO. 50, or it is a sequence variant having at least 80% homology to the sequence set forth in SEQ ID NO. 50.

In the complementary determining regions of the heavy chain variable region mentioned above, the sequences set forth in SEQ ID NOs. 27-50 are the CDR sequences of camelid-derived antibodies, and the sequences set forth in SEQ ID NOs. 51-58 are the CDR sequences of humanized antibodies. In the present invention, experiments have verified that the CDR sequences of the camelid-derived antibodies and the CDR sequences of the humanized antibodies have high affinity for IL-18R1 and IL-18RAP, and can efficiently bind to IL-18R1 and IL-18RAP.

For the sequence variants mentioned, the above CDR sequences are mutated by a histidine scanning mutagenesis in the present application, resulting in sequence variants that can bind to IL-18R1 and IL-18RAP with high affinity. These sequence variants have at least 80% homology compared to the original sequence before mutation.

Preferably, in the above (1) - (8), the sequence variant having at least 80% homology isa sequence obtained by substituting any one amino acid of the original sequence with histidine. The original sequence is any sequence set forth in SEQ ID NOs. 27-58.

For example, for the sequence variant corresponding to CDR1 in (1) above, its original sequence refers to the sequence set forth in SEQ ID NO. 27. That is, one amino acid in the sequence set forth in SEQ ID NO. 27 is substituted with histidine, to obtain the sequence variant. This variant still has a high affinity for IL-18R1.

Preferably, a total of only one amino acid in the amino acid sequence of CDR1, CDR2, and CDR3 of each antibody or the antigen-binding fragment thereof is substituted with histidine.

For example, for the complementary determining regions (CDR1, CDR2, and CDR3) shown in (1), the histidine mutation of their sequence variants only occurs at one amino acid position within one of the amino acid sequences selected from SEQ ID NOs. 27-29 and 51.

The sequence variants obtained by the histidine scanning mutagenesis mentioned above are merely illustrative. Those skilled in the art can modify the CDRs described above using "conservative sequence modification" to obtain different sequence variants. "Conservative sequence modification" refers to an amino acid sequence modification that does not significantly affect or alter the binding characteristics of the antibody or the antigen-binding fragment thereof, including amino acid substitution, addition, or deletion. Modification can be introduced into the sequences set forth in SEQ ID NOs. 27-58 using conventional techniques in the art, such as site-directed mutagenesis and PCR-mediated mutagenesis. Conservative amino acid substitution includes substituting an amino acid residue with an amino acid residue having a similar side chain or other amino acid residues. Families of amino acid residues with similar side chains have been defined in the art. These families include amino acids with basic side chains (such as lysine, arginine, histidine), amino acids with acidic side chains (such as aspartic acid, glutamic acid), amino acids with uncharged polar side chains (such as glycine, asparagine, glutamine, serine, threonine, tyrosine, cysteine, tryptophan), amino acids with non-polar side chains (such as alanine, valine, leucine, isoleucine, proline, phenylalanine, methionine), amino acids with β-branched side chains (such as threonine, valine, isoleucine), and amino acids with aromatic side chains (such as tyrosine, phenylalanine, tryptophan, histidine), and the like.

Preferably, the amino acid sequence of the heavy chain variable region of the antibody or the antigen-binding fragment thereof that binds to IL-18R1 is set forth in any one of SEQ ID NOs. 7-11, or set forth in any one of SEQ ID NOs. 20-23, or it is a sequence variant having at least 80% homology to any one of SEQ ID NOs. 7-11 and SEQ ID NO. 20-23.

In an embodiment, the sequences set forth in SEQ ID NOs. 7-11 are the sequences of the heavy chain variable regions of camelid-derived antibodies, and the sequences set forth in SEQ ID NOs. 20-23 are the sequences of the heavy chain variable regions of humanized antibodies. In the present invention, experiments have verified that both the heavy chain variable regions of the camelid-derived antibodies and the heavy chain variable regions of the humanized antibodies have high affinity for IL-18R1 and can efficiently bind to IL-18R1.

Preferably, the amino acid sequence of the heavy chain variable region of the antibody or the antigen-binding fragment thereof that binds to IL-18RAP is set forth in any one of SEQ ID NOs. 12-14, or set forth in any one of SEQ ID NOs. 24-26, or it is a sequence variant having at least 80% homology to any one of SEQ ID NOs. 12-14 and SEQ ID NOs. 24-26.

In an embodiment, the sequences set forth in SEQ ID NOs. 12-14 are the sequences of the heavy chain variable regions of camelid-derived antibodies, and the sequences set forth in SEQ ID NOs. 24-26 are the sequences of the heavy chain variable regions of humanized antibodies. In the present invention, experiments have verified that both the heavy chain variable region of the camelid-derived antibody and the heavy chain variable region of the humanized antibody have high affinity for IL-18RAP and can efficiently bind to IL-18RAP.

Among the above-mentioned heavy chain variable regions, the sequence variant having at least 80% homology is a sequence obtained by substituting a total of one amino acid in CDR1, CDR2, and CDR3 of any one of the sequences set forth in SEQ ID NOs. 7-14 and SEQ ID NOs. 20-26 with histidine, that is, only one amino acid position of one CDR among CDR1, CDR2, and CDR3 in each heavy chain variable region is mutated to histidine.

Preferably, for the sequence set forth in SEQ ID NO. 7 or SEQ ID NO. 20, the sequence variant having at least 80% homology is a sequence obtained by substituting the amino acid at position 27, 28, 29, 30, 31, 32, 34, 35, 50, 51, 53, 54, 55, 56, 57, 58, 59, 60, 61, 62, 63, 64, 65, 98, 99, 100, 102, 103, 104, 105, 106, 107, 108, 109 or 110 of the sequence set forth in SEQ ID NO. 7 or SEQ ID NO. 20 with histidine.

Preferably, for the sequence set forth in SEQ ID NO. 10 or SEQ ID NO. 23, a sequence variant having at least 80% homology is a sequence obtained by substituting the amino acid at position 31, 32, 33, 48, 49, 50, 51, 52, 53, 54, 55, 56, 57, 58, 59, 60, 61, 62, 63, 96, 97, 98, 99, 100, 101, 102, 103, 104, 105, 107, 108, 109, 110, 111, 112 or 29 of the sequence set forth in SEQ ID NO. 10 or SEQ ID NO. 23 with histidine, or by substituting the amino acids at positions 52 and 59, 52 and 105, 52 and 107, 59 and 99, 59 and 105, 59 and 107, 59 and 109, 99 and 107, 105 and 109, 52 and 112, 59 and 112, 101 and 112, or 105 and 112 of the sequence set forth in SEQ ID NO. 10 or SEQ ID NO. 23 with histidine.

Preferably, for the sequence set forth in SEQ ID NO. 13 or SEQ ID NO. 25, a sequence variant having at least 80% homology is a sequence a sequence obtained by substituting the amino acid at position 161, 162, 163, 164, 179, 180, 181, 182, 183, 184, 185, 186, 187, 188, 189, 190, 191, 192, 193, 194, 195, 228, 229, 230, 231, 232, 233, 234 or 235 of the sequence set forth in SEQ ID NO. 13 or SEQ ID NO. 25 with histidine.

The sequence variants obtained by the histidine scanning mutagenesis mentioned above are merely illustrative. Those skilled in the art can modify heavy chain variable regions described above using techniques such as "conservative sequence modification" to obtain other different sequence variants.

In the present application, the antibody or the antigen-binding fragment thereof is a single-domain antibody, a minibody, a bispecific antibody, a multispecific antibody, a monoclonal antibody, a single-chain antibody, Fab, Fab', F(ab')₂, Fd, Fv, scFv, BsFv, dsFv, or (dsFv)₂.

In some embodiments of the present application, the antibody is a single-domain antibody.

In some embodiments of the present application, the antibody is a bispecific antibody containing the single-domain antibody.

In the present application, the antibody or the antigen-binding fragment thereof is a camelid-derived antibody, a humanized antibody, a murine-derived antibody, a rabbit-derived antibody, or a chimeric antibody.

In some embodiments of the present application, the antibody is a camelid-derived antibody.

In some embodiments of the present application, the antibody is a humanized antibody.

In the present application, in addition to the aforementioned heavy chain variable region, the antibody or the antigen-binding fragment thereof that binds to an IL-18 receptor may further have one or more sequences selected from a protein tag, an enzymatic cleavage site, and a linker peptide connected to the N-terminus and/or C-terminus of the heavy chain variable region. The connection of these sequences will not significantly affect the function of the antibody or the antigen-binding fragment thereof.

There are no special limitations on the sequences of the protein tag, the enzymatic cleavage site, and the linker peptide. Protein tags include but are not limited to His-tag, Gst, MBP, Strep, Flag, and the like. Enzymatic cleavage sites can be enzymes for cleaving protein tags, including but not limited to TEV protease, Sortase (transpeptidase), and the like. Linker peptides can be short peptides rich in glycine and serine.

In a second aspect, there is provided in the present application a bispecific antibody comprising the antibody or the antigen-binding fragment thereof that binds to an IL-18 receptor as described above.

Specifically, the bispecific antibody may include the antibody or the antigen-binding fragment thereof that binds to IL-18R1 as described above, as well as the antibody or the antigen-binding fragment thereof that binds to IL-18RAP as described above or the antibody or the antigen-binding fragment thereof that binds to IL-18RAP that is not covered by the present application (such as IL-18RAP antibodies known in the prior art).

Similarly, the bispecific antibodies may also include the antibody or the antigen-binding fragment thereof that binds to IL-18RAP as described above, as well as the antibody or the antigen-binding fragment thereof that binds to IL-18R1 that is not covered by the present application (such as IL-18R1 antibodies known in the prior art).

In addition, the bispecific antibody may also include the antibody or the antigen-binding fragment thereof that binds to IL-18R1 or IL-18RAP as described above, as well as the antibody with other functions (non-IL-18 receptor binding function).

In some embodiments of the present application, the bispecific antibody includes one or more antibodies or antigen-binding fragments thereof that bind to IL-18R1 as described above and one or more antibodies or antigen-binding fragments thereof that bind to IL-18RAP as described above.

Preferably, in the bispecific antibody, the amino acid sequence of the antibody or the antigen-binding fragment thereof that binds to IL-18R1 is set forth in SEQ ID NO. 7, and the amino acid sequence of the antibody or the antigen-binding fragment thereof that binds to IL-18RAP is set forth in SEQ ID NO. 12; or
the amino acid sequence of the antibody or the antigen-binding fragment thereof that binds to IL-18R1 is set forth in SEQ ID NO. 8, and the amino acid sequence of the antibody or the antigen-binding fragment thereof that binds to IL-18RAP is set forth in SEQ ID NO. 14; or
the amino acid sequence of the antibody or the antigen-binding fragment thereof that binds to IL-18R1 is set forth in SEQ ID NO. 8, and the amino acid sequence of the antibody or the antigen-binding fragment thereof that binds to IL-18RAP is set forth in SEQ ID NO. 13; or
the amino acid sequence of the antibody or the antigen-binding fragment thereof that binds to IL-18R1 is set forth in SEQ ID NO. 9, and the amino acid sequence of the antibody or the antigen-binding fragment thereof that binds to IL-18RAP is set forth in SEQ ID NO. 14; or
the amino acid sequence of the antibody or the antigen-binding fragment thereof that binds to IL-18R1 is set forth in SEQ ID NO. 9, and the amino acid sequence of the antibody or the antigen-binding fragment thereof that binds to IL-18RAP is set forth in SEQ ID NO. 13; or
the amino acid sequence of the antibody or the antigen-binding fragment thereof that binds to IL-18R1 is set forth in SEQ ID NO. 10, and the amino acid sequence of the antibody or the antigen-binding fragment thereof that binds to IL-18RAP is set forth in SEQ ID NO. 13; or
the amino acid sequence of the antibody or the antigen-binding fragment thereof that binds to IL-18R1 is set forth in SEQ ID NO. 11, and the amino acid sequence of the antibody or the antigen-binding fragment thereof that binds to IL-18RAP is set forth in SEQ ID NO. 12; or
the amino acid sequence of the antibody or the antigen-binding fragment thereof that binds to IL-18R1 is set forth in SEQ ID NO. 20, and the amino acid sequence of the antibody or the antigen-binding fragment thereof that binds to IL-18RAP is set forth in SEQ ID NO. 24; or
the amino acid sequence of the antibody or the antigen-binding fragment thereof that binds to IL-18R1 is set forth in SEQ ID NO. 21, and the amino acid sequence of the antibody or the antigen-binding fragment thereof that binds to IL-18RAP is set forth in SEQ ID NO. 26; or
the amino acid sequence of the antibody or the antigen-binding fragment thereof that binds to IL-18R1 is set forth in SEQ ID NO. 21, and the amino acid sequence of the antibody or the antigen-binding fragment thereof that binds to IL-18RAP is set forth in SEQ ID NO. 25; or
the amino acid sequence of the antibody or the antigen-binding fragment thereof that binds to IL-18R1 is set forth in SEQ ID NO. 22, and the amino acid sequence of the antibody or the antigen-binding fragment thereof that binds to IL-18RAP is set forth in SEQ ID NO. 26; or
the amino acid sequence of the antibody or the antigen-binding fragment thereof that binds to IL-18R1 is set forth in SEQ ID NO. 22, and the amino acid sequence of the antibody or the antigen-binding fragment thereof that binds to IL-18RAP is set forth in SEQ ID NO. 25; or
the amino acid sequence of the antibody or the antigen-binding fragment thereof that binds to IL-18R1 is set forth in SEQ ID NO. 23, and the amino acid sequence of the antibody or the antigen-binding fragment thereof that binds to IL-18RAP is set forth in SEQ ID NO. 25; or
the amino acid sequence of the antibody or the antigen-binding fragment thereof that binds to IL-18R1 is set forth in SEQ ID NO. 11, and the amino acid sequence of the antibody or the antigen-binding fragment thereof that binds to IL-18RAP is set forth in SEQ ID NO. 24; or
the amino acid sequence of the antibody or the antigen-binding fragment thereof that binds to IL-18R1 is a sequence obtained by substituting the amino acid at position 27, 28, 29, 30, 31, 32, 34, 35, 50, 51, 53, 54, 55, 56, 57, 58, 59, 60, 61, 62, 63, 64, 65, 98, 99, 100, 102, 103, 104, 105, 106, 107, 108, 109 or 110 of the sequence set forth in SEQ ID NO. 20 with histidine, and the amino acid sequence of the antibody or the antigen-binding fragment thereof that binds to IL-18RAP is set forth in SEQ ID NO. 24; or
the amino acid sequence of the antibody or the antigen-binding fragment thereof that binds to IL-18R1 is a sequence obtained by substituting the amino acid at position 31, 32, 33, 48, 49, 50, 51, 52, 53, 54, 55, 56, 57, 58, 59, 60, 61, 62, 63, 96, 97, 98, 99, 100, 101, 102, 103, 104, 105, 107, 108, 109, 110, 111, 112 or 29 of the sequence set forth in SEQ ID NO. 23 with histidine, or by substituting the amino acids at positions 52 and 59, 52 and 105, 52 and 107, 59 and 99, 59 and 105, 59 and 107, 59 and 109, 99 and 107, 105 and 109, 52 and 112, 59 and 112, 101 and 112, or 105 and 112 of the sequence set forth in SEQ ID NO. 23 with histidine; and the amino acid sequence of the antibody or the antigen-binding fragment thereof that binds to IL-18RAP is set forth in SEQ ID NO. 25; or
the amino acid sequence of the antibody or the antigen-binding fragment thereof that binds to IL-18R1 is set forth in SEQ ID NO. 23, and the amino acid sequence of the antibody or the antigen-binding fragment thereof that binds to IL-18RAP is a sequence obtained by substituting the amino acid at position 161, 162, 163, 164, 179, 180, 181, 182, 183, 184, 185, 186, 187, 188, 189, 190, 191, 192, 193, 194, 195, 228, 229, 230, 231, 232, 233, 234 or 235 of the sequence set forth in SEQ ID NO. 25 with histidine.

Preferably, in the bispecific antibodies described above, the antibody or the antigen-binding fragment thereof that binds to IL-18R1 is linked (in tandem) with the antibody or the antigen-binding fragment thereof that binds to IL-18RAP.

The linkage can be configured in an orientation wherein the antibody or the antigen-binding fragment thereof that binds to IL-18R1 is at the N-terminus, the antibody or the antigen-binding fragment thereof that binds to IL-18RAP is at the C-terminus, or in an orientation wherein the antibody or the antigen-binding fragment thereof that binds to IL-18RAP is at the N-terminus and the antibody or the antigen-binding fragment thereof that binds to IL-18R1 is at the C-terminus.

Preferably, the antibody or the antigen-binding fragment thereof that binds to IL-18R1 is linked to the antibody or the antigen-binding fragment that binds to IL-18RAP via a linker. The linker may be any flexible peptide or linker peptide, such as G4S or a similar linker peptide or flexible peptide of other lengths and amino acid compositions.

In some embodiments of the present application, G4S flexible linker (SEQ ID NO. 15), GS flexible linker, (G4S)2 flexible linker (SEQ ID NO. 17), (G4S)3 flexible linker (SEQ ID NO. 18), (G4S)4 flexible linker (SEQ ID NO. 19), GGGGSG (SEQ ID NO. 66), GGGSGG (SEQ ID NO. 67), GGSGGG (SEQ ID NO. 68), GSGGGG (SEQ ID NO. 69), GGSGSG (SEQ ID NO. 70) or GSGSGG (SEQ ID NO. 71) may be used to link the antibody or the antigen-binding fragment thereof that binds to IL-18R1 with the antibody or the antigen-binding fragment thereof that binds to IL-18RAP.

Preferably, the bispecific antibody includes any one of the following domains (1) - (21):
(1) the amino acid sequence arranged sequentially from the N-terminus to the C-terminus as follows: the sequence set forth in SEQ ID NO. 7, a linker, and the sequence set forth in SEQ ID NO. 12;
(2) the amino acid sequence arranged sequentially from the N-terminus to the C-terminus as follows: the sequence set forth in SEQ ID NO. 8, a linker, and the sequence set forth in SEQ ID NO. 14;
(3) the amino acid sequence arranged sequentially from the N-terminus to the C-terminus as follows: the sequence set forth in SEQ ID NO. 8, a linker, and the sequence set forth in SEQ ID NO. 13;
(4) the amino acid sequence arranged sequentially from the N-terminus to the C-terminus as follows: the sequence set forth in SEQ ID NO. 9, a linker, and the sequence set forth in SEQ ID NO. 14;
(5) the amino acid sequence arranged sequentially from the N-terminus to the C-terminus as follows: the sequence set forth in SEQ ID NO. 9, a linker, and the sequence set forth in SEQ ID NO. 13;
(6) the amino acid sequence arranged sequentially from the N-terminus to the C-terminus as follows: the sequence set forth in SEQ ID NO. 10, a linker, and the sequence set forth in SEQ ID NO. 13;
(7) the amino acid sequence arranged sequentially from the N-terminus to the C-terminus as follows: the sequence set forth in SEQ ID NO. 12, a linker, and the sequence set forth in SEQ ID NO. 7;
(8) the amino acid sequence arranged sequentially from the N-terminus to the C-terminus as follows: the sequence set forth in SEQ ID NO. 13, a linker, and the sequence set forth in SEQ ID NO. 9;
(9) the amino acid sequence arranged sequentially from the N-terminus to the C-terminus as follows: the sequence set forth in SEQ ID NO. 13, a linker, and the sequence set forth in SEQ ID NO. 10;
(10) the amino acid sequence arranged sequentially from the N-terminus to the C-terminus as follows: the sequence set forth in SEQ ID NO. 12, a linker, and the sequence set forth in SEQ ID NO. 11;
(11) the amino acid sequence arranged sequentially from the N-terminus to the C-terminus as follows: the sequence set forth in SEQ ID NO. 20, a linker, and the sequence set forth in SEQ ID NO. 24;
(12) the amino acid sequence arranged sequentially from the N-terminus to the C-terminus as follows: the sequence set forth in SEQ ID NO. 21, a linker, and the sequence set forth in SEQ ID NO. 26;
(13) the amino acid sequence arranged sequentially from the N-terminus to the C-terminus as follows: the sequence set forth in SEQ ID NO. 21, a linker, and the sequence set forth in SEQ ID NO.25;
(14) the amino acid sequence arranged sequentially from the N-terminus to the C-terminus as follows: the sequence set forth in SEQ ID NO. 22, a linker, and the sequence set forth in SEQ ID NO. 26;
(15) the amino acid sequence arranged sequentially from the N-terminus to the C-terminus as follows: the sequence set forth in SEQ ID NO. 22, a linker, and the sequence set forth in SEQ ID NO. 25;
(16) the amino acid sequence arranged sequentially from the N-terminus to the C-terminus as follows: the sequence set forth in SEQ ID NO. 23, a linker, and the sequence set forth in SEQ ID NO. 25;
(17) the amino acid sequence arranged sequentially from the N-terminus to the C-terminus as follows: the sequence set forth in SEQ ID NO. 24, a linker, and the sequence set forth in SEQ ID NO. 20;
(18) the amino acid sequence arranged sequentially from the N-terminus to the C-terminus as follows: the sequence set forth in SEQ ID NO. 25, a linker, and the sequence set forth in SEQ ID NO. 22;
(19) the amino acid sequence arranged sequentially from the N-terminus to the C-terminus as follows: the sequence set forth in SEQ ID NO. 25, a linker, and the sequence set forth in SEQ ID NO. 23;
(20) the amino acid sequence arranged sequentially from the N-terminus to the C-terminus as follows: the sequence set forth in SEQ ID NO. 24, a linker, and the sequence set forth in SEQ ID NO. 11;
(21) the amino acid sequence obtained by substituting the amino acid at position 27, 28, 29, 30, 31, 32, 34, 35, 50, 51, 53, 54, 55, 56, 57, 58, 59, 60, 61, 62, 63, 64, 65, 98, 99, 100, 102, 103, 104, 105, 106, 107, 108, 109 or 110 of the sequence set forth in SEQ ID NO. 20 with histidine on the basis of the amino acid arranged sequentially from the N-terminus to the C-terminus as follows: the sequence set forth in; SEQ ID NO. 20, a linker, and the sequence set forth in SEQ ID NO. 24;
(22) the amino acid sequence arranged sequentially from the N-terminus to the C-terminus as follows: a sequence obtained by substituting the amino acids at positions 31, 32, 33, 48, 49, 50, 51, 52, 53, 54, 55, 56, 57, 58, 59, 60, 61, 62, 63, 96, 97, 98, 99, 100, 101, 102, 103, 104, 105, 107, 108, 109, 110, 111, 112 or 29 of the sequence set forth in SEQ ID NO. 23 with histidine, or obtained by substituting the amino acids at positions 52 and 59, 52 and 105, 52 and 107, 59 and 99, 59 and 105, 59 and 107, 59 and 109, 99 and 107, 105 and 109, 52 and 112, 59 and 112, 101 and 112, or 105 and 112 of the sequence set forth in SEQ ID NO. 23 with histidine, a linker, and the sequence set forth in SEQ ID NO. 25; and
(23) the amino acid sequence arranged sequentially from the N-terminus to the C-terminus as follows: the sequence set forth in SEQ ID NO. 23, a linker, and a sequence obtained by substituting the amino acid at position 161, 162, 163, 164, 179, 180, 181, 182, 183, 184, 185, 186, 187, 188, 189, 190, 191, 192, 193, 194, 195, 228, 229, 230, 231, 232, 233, 234 or 235 of the sequence set forth in SEQ ID NO. 25.

The above (1) - (10) are camelid-derived bispecific antibodies, and (11) - (23) are humanized bispecific antibodies. The humanized bispecific antibody of (21) is a variant of the humanized bispecific antibody of (11) obtained by the histidine scanning mutagenesis. The humanized bispecific antibody of (22) is a variant of the humanized bispecific antibody of (16) obtained by the histidine scanning mutagenesis. The humanized bispecific antibody of (23) is a variant of the humanized bispecific antibody of (16) obtained by the histidine scanning mutagenesis. In the present invention, experiments have verified that these variants all maintain good immunocyte-activating activity.

In the case where the antibody or the antigen-binding fragment thereof that binds to IL-18R1 and the antibody or the antigen-binding fragment thereof that binds to IL-18RAP are included, in the present application, there is no special limitation on the structure of the bispecific antibody, and it can be designed in any structure currently known.

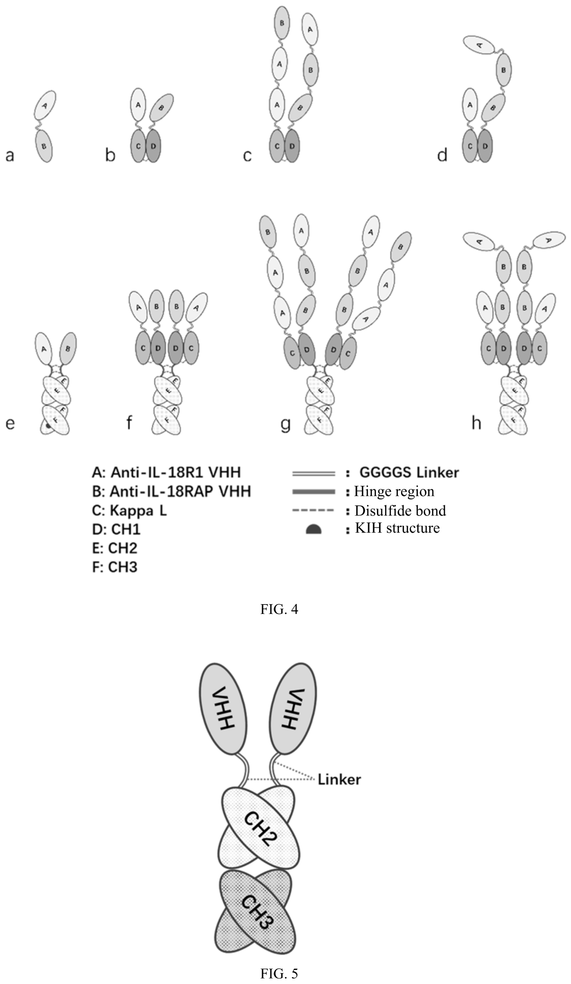

In some embodiments of the present application, the bispecific antibody further includes any one or more selected from KappaL, CH1, CH2, CH3, hinge region, and KIH structure. Alternatively, the bispecific antibody structure is set forth in FIG. 4.

In a third aspect, there is provided in the present application a fusion protein, including the antibody or the antigen-binding fragment thereof that binds to the IL-18 receptor, or including the bispecific antibody.

Preferably, the fusion protein is obtained by fusing the antibody or the antigen-binding fragment thereof that binds to the IL-18 receptor or the bispecific antibody with other proteins.

As an example, for the purpose of facilitating preparation, improving molecular stability and pharmacokinetic characteristics in vivo, and the like, the antibody or the antigen-binding fragment thereof that binds to the IL-18 receptor or the bispecific antibody may be fused with proteins such as Fab, Fc, HAS, to form fusion proteins. For the purpose of further enhancing drug efficacy, the antibody or the antigen-binding fragment thereof that binds to the IL-18 receptor or the bispecific antibody is fused with other cytokines and antibodies (such as aPD-L1, aPD-1, and the like) to form fusion proteins.

Alternatively, said other protein includes one or more selected from protein tag, Fab, Fc, HSA, cytokine, or antibody.

In some embodiments of the present application, the antibody or the antigen-binding fragment thereof that binds to the IL-18 receptor or the bispecific antibody is fused with Fc to obtain a fusion protein. Fc may be a human Fc tag (hFc, SEQ ID NO. 1), a mouse Fc tag (mFc, SEQ ID NO. 2), or an camelid-derived Fc tag (aFc, SEQ ID NO. 3).

In a fourth aspect, there is provided in the present application a bifunctional protein, including the antibody or the antigen-binding fragment thereof that binds to the IL-18 receptor, or the bispecific antibody as described above, and further including a targeting protein, an antibody, or a cytokine.

Preferably, the antibody is an immune checkpoint antibody and/or a tumor-targeted antibody.

In a fifth aspect, the present application provides a nucleic acid molecule encoding the antibody or the antigen-binding fragment thereof that binds to the IL-18 receptor, or the bispecific antibody, or fusion protein, or bifunctional protein as described above.

Based on the amino acid sequences of the antibody or the antigen-binding fragment thereof that binds to the IL-18 receptor, the bispecific antibody, the fusion protein, and the bifunctional protein as described above, those skilled in the art can obtain the nucleotide sequences of nucleic acid molecules encoding the antibody or the antigen-binding fragment thereof that binds to the IL-18 receptor, the bispecific antibody, the fusion protein, and the bifunctional protein as described above. Due to the degeneracy of codons, the nucleotide sequences of nucleic acid molecules encoding the same protein are not unique. All nucleic acid molecules that can encode the antibody or the antigen-binding fragment thereof that binds to the IL-18 receptor, the bispecific antibody, the fusion protein, and the bifunctional protein are within the protection scope of the present application.

In a sixth aspect, there is provided in the present application a biological material, including an expression cassette, a vector, or a host cell containing the aforementioned nucleic acid molecules.

Wherein, the expression cassette is a recombinant nucleic acid molecule obtained by linking the nucleic acid molecule with regulatory elements for transcription, translation, and the like.

The vector includes but is not limited to a plasmid vector, a phage vector, a viral vector, an artificial chromosome vector, and the like.

The host cell includes a microbial cell (such as *Escherichia coli,* yeast, and the like), an insect cell, or other animal cell.

In a seventh aspect, there is provided in the present application a method for preparing the antibody or the antigen-binding fragment thereof that binds to the IL-18 receptor, the bispecific antibody, the fusion protein, or the bifunctional protein. The method includes: introducing a nucleic acid molecule encoding the antibody or the antigen-binding fragment thereof that binds to the IL-18 receptor, the bispecific antibody, the fusion protein, or the bifunctional protein into a host cell to obtain a recombinant host cell; cultivating the recombinant host cell, and isolating and obtaining the antibody or the antigen-binding fragment thereof that binds to the IL-18 receptor, or the bispecific antibody or the fusion protein.

The above-mentioned host cell includes but is not limited to a microbial cell (such as *Escherichia coli,* yeast, and the like), an insect cell, or other animal cell.

In an eighth aspect, there is provided in the present application any of the following uses of the antibody or the antigen-binding fragment thereof that binds to the IL-18 receptor, the bispecific antibody, the fusion protein, the bifunctional protein, the nucleic acid molecule, or the biological material:
(1) use in the preparation of anti-tumor drugs;
(2) use in the preparation of drugs that enhance a body's immune response;
(3) use in the preparation of drugs for activating IL-18 signaling pathway;
(4) use in the preparation of drugs for stimulating the secretion of INF γ and/or GZMB;
(5) use in the preparation of IL-18 receptor activators;
(6) use in the preparation of drugs for the prevention or treatment of IL-18 receptor related diseases; and
(7) use in the preparation of reagents for detecting IL-18 receptor content.

In the above (1), the tumors include all tumors that can be treated with immunotherapy, including but not limited to digestive system tumors (such as cholangiocarcinoma, gastric cancer, pancreatic cancer, intestinal cancer, and the like), respiratory system tumors (such as squamous lung cancer, non-small cell lung cancer, small cell lung cancer, and the like), urinary system tumors (bladder cancer, urothelial cancer and the like), nervous system tumors, reproductive system tumors (ovarian cancer, breast cancer, cervical cancer, and the like), melanoma, and the like.

In a ninth aspect, there is provided an IL-18 receptor activator according to the present application, including the antibody or the antigen-binding fragment thereof that binds to the IL-18 receptor, the bispecific antibody, the fusion protein, or the bifunctional protein as described above.

In a tenth aspect, there is provided a pharmaceutical composition according to the present application, including the antibody or the antigen-binding fragment thereof that binds to the IL-18 receptor, the bispecific antibody, the fusion protein, or the bifunctional protein as described above.

In an eleventh aspect, there is provided a detection reagent including the antibody or the antigen-binding fragment thereof that binds to the IL-18 receptor, the bispecific antibody, the fusion protein, or the bifunctional protein as described above according to the present application.

In the present application, the preferred IL-18 is human IL-18.

The beneficial effect of the present application is that it provides an antibody that specifically binds to IL-18R1 and an antibody that specifically binds to IL-18RAP. These antibodies have high affinity for IL-18R1 and IL-18RAP. These antibodies are further tandem to form bispecific antibodies that can efficiently bind to IL-18R1 and IL-18RAP (IL-18R α/β heterodimer complex) simultaneously. These bispecific antibodies can activate the intracellular signaling pathway mediated by IL-18R1/IL-18RAP that activates the IL-18 signaling pathway. These bispecific antibodies can exert IL-18-like biological functions both in vitro and in vivo, exhibiting a relatively broad-spectrum immune enhancement effect, and stimulating the proliferation and activation of NK cells and T cells, as well as their secretion of cytokines such as IFN-γ and the like. The tumor growth is significantly inhibited in vivo experiments of animal models, and it has broad clinical application prospects for anti-tumor treatment.

The bispecific antibody provided by the present application is a novel form of IL-18 receptor activator that can avoid the negative feedback of IL-18 pathway activation caused by IL-18BP antagonism, and has controllable activation activity, better molecular stability, and in vivo pharmacokinetic characteristics, which are more conducive to fully exerting anti-tumor efficacy in vivo.

### BRIEF DESCRIPTION OF THE DRAWINGS

To illustrate the embodiments of the present application or the prior art more clearly, a brief introduction will be given to the accompanying drawings required for the embodiments or the description of the prior art. It is obvious that the accompanying drawings described below are some embodiments of the present application. For those skilled in the art, other drawings can be obtained based on these drawings without creative labor.

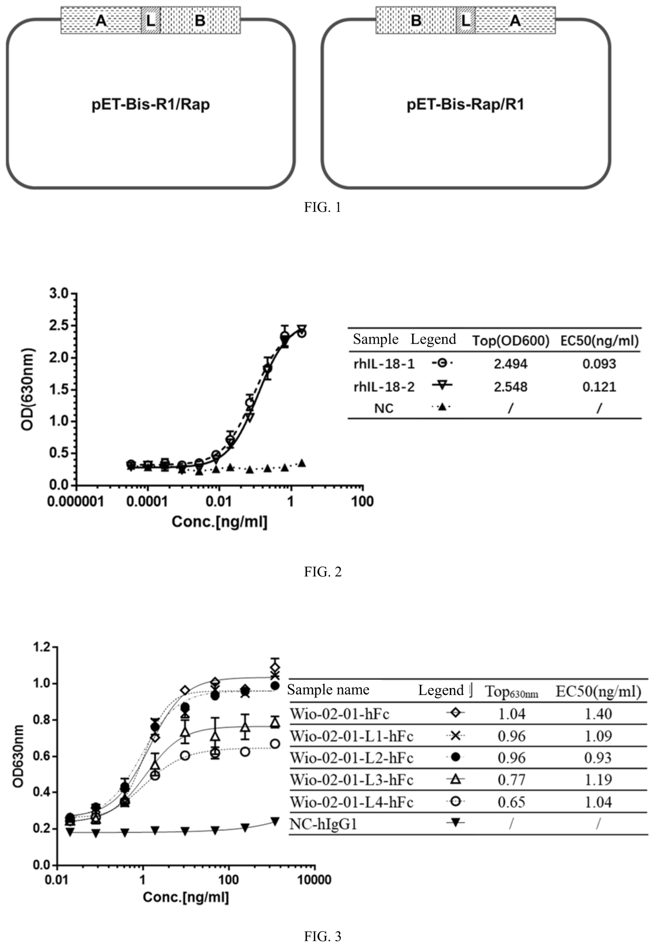

FIG. 1 is a schematic diagram of the construction of a prokaryotic expression vector for an anti-human IL-18R1/RAP forward/reverse bispecific antibody in Example 3 according to the present application, where A represents Anti-IL-18R1 VHH; B represents Anti-IL-18RAP VHH; and L represents a GGGGS linker.
FIG. 2 is a graph showing the results of detecting rhIL-18 activity using the reporter gene system in Example 5 according to the present application, where rhIL-18-1 and rhIL-18-2 represent two independent repeated experiments.
FIG. 3 is a graph showing the effect of linkers with different lengths on the activity of Wio-02-01 in Example 6 according to the present application.
FIG. 4 is a schematic structural diagram of the anti-human IL-18R1/RAP bispecific antibody in Example 7 according to the present application.
FIG. 5 is a schematic structural diagram of the recombinant anti-human IL-18R1 or anti-human IL-18RAP camelid-derived heavy chain single-domain antibody in Example 8 according to the present application.

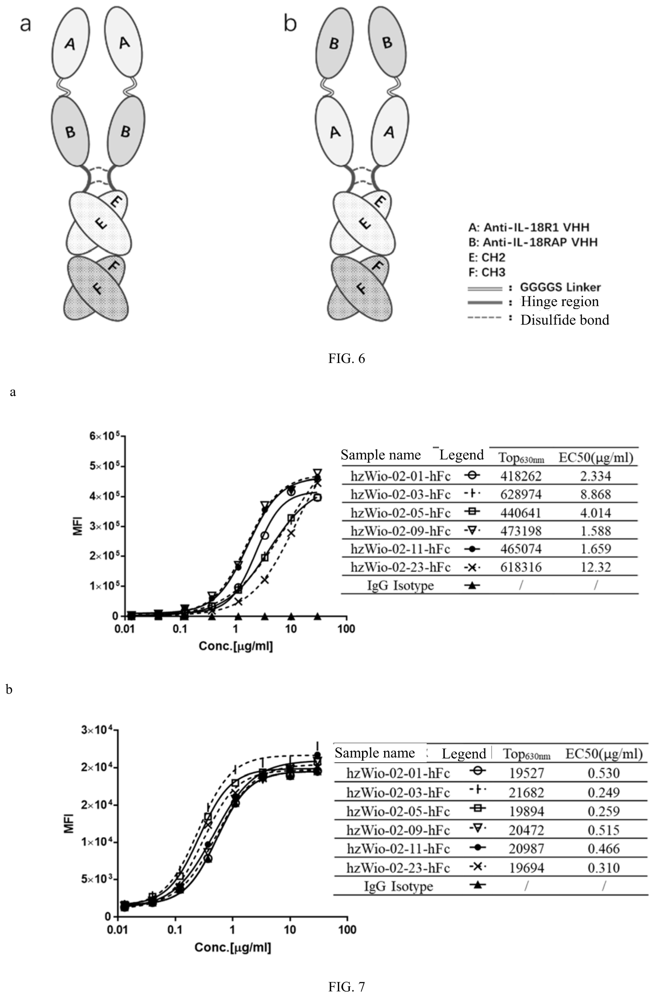

FIG. 6 is a schematic structural diagram of the Fc fusion protein of the anti-human IL-18R1/RAP bispecific antibody in Example 9 according to the present application, where a is a humanized bispecific antibody with a forward structure; b is a humanized bispecific antibody with a reverse structure.
FIG. 7 is a graph showing the binding activity of the anti-human IL-18R1/RAP bispecific antibody to IL-18R1 and IL-18RAP on the cell membrane surface in Example 12 according to the present application, where a shows the binding activity of the anti-human IL-18R1/RAP bispecific antibody to IL-18R1 on the cell membrane surface at antibody concentrations of 30, 10, 3.33, 1.11, 0.37, 0.12, 0.041, and 0.014 µg/ml, and b shows the binding activity of the anti-human IL-18R1/RAP bispecific antibody to IL-18RAP on the cell membrane surface at antibody concentrations of 30, 10, 3.33, 1.11, 0.37, 0.12, 0.041, and 0.014 µg/ml.

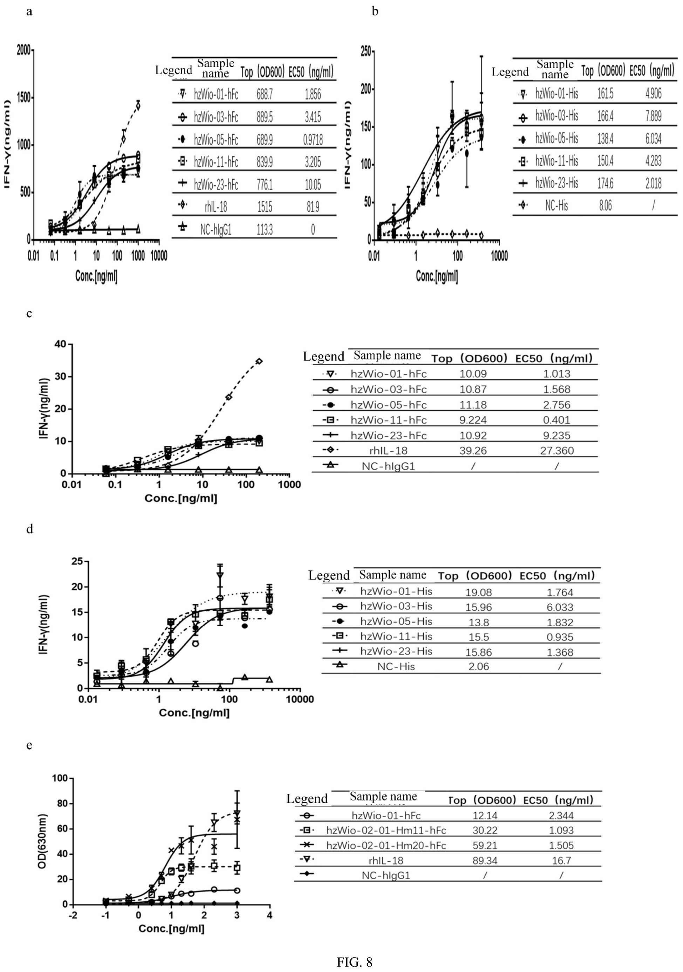

FIG. 8 is a graph showing the activity evaluation of the humanized anti-IL-18R1/RAP bispecific antibody in Example 12 according to the present application through a PBMC activation experiment, where a is a graph showing the activity evaluation of the humanized anti-IL-18R1/RAP bispecific antibody (linked to hFc) through a PBMC activation experiment (+IL-12), b is a graph showing the activity evaluation of the humanized anti-IL-18R1/RAP bispecific antibody (linked to a histidine tag) through a PBMC activation experiment (+IL-12), c is a graph showing the activity evaluation of the humanized anti-IL-18R1/RAP bispecific antibody (linked to hFc) through a PBMC activation experiment (+IL-15), d is a graph showing the activity evaluation of the humanized anti-IL-18R1/RAP bispecific antibody (linked to a histidine tag) through a PBMC activation experiment (+IL-15) and e is a graph showing the activity evaluation of the humanized anti-IL-18R1/RAP bispecific antibody (mutant) through a PBMC activation experiment (+IL-15).

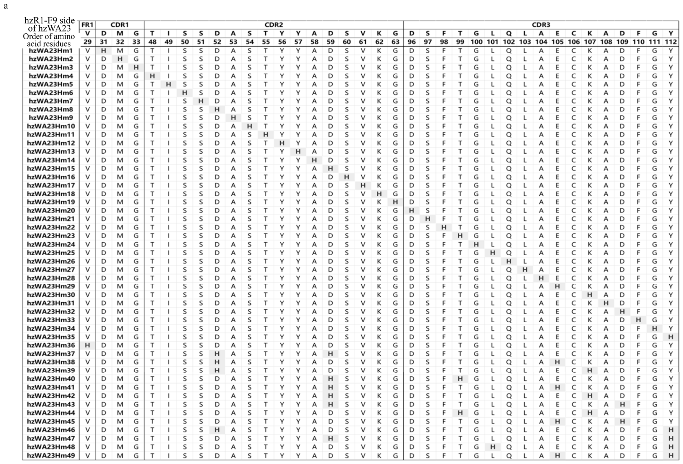

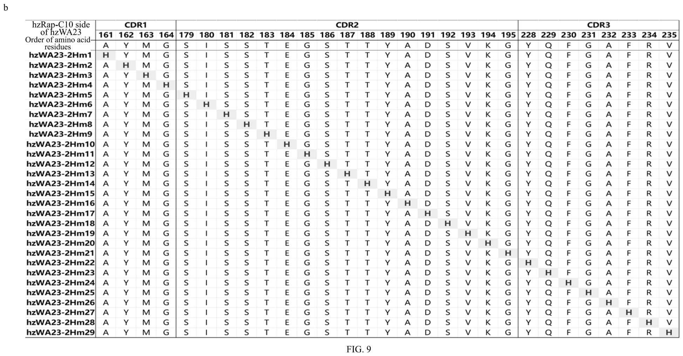

FIG. 9 shows the list of histidine scanning mutants of hzWA23 in Example 13 according to the present application, where a shows the mutants on the hzR1-F9 side of hzWA23; b shows the mutants on the hzRAP-C10 side of hzWA23.

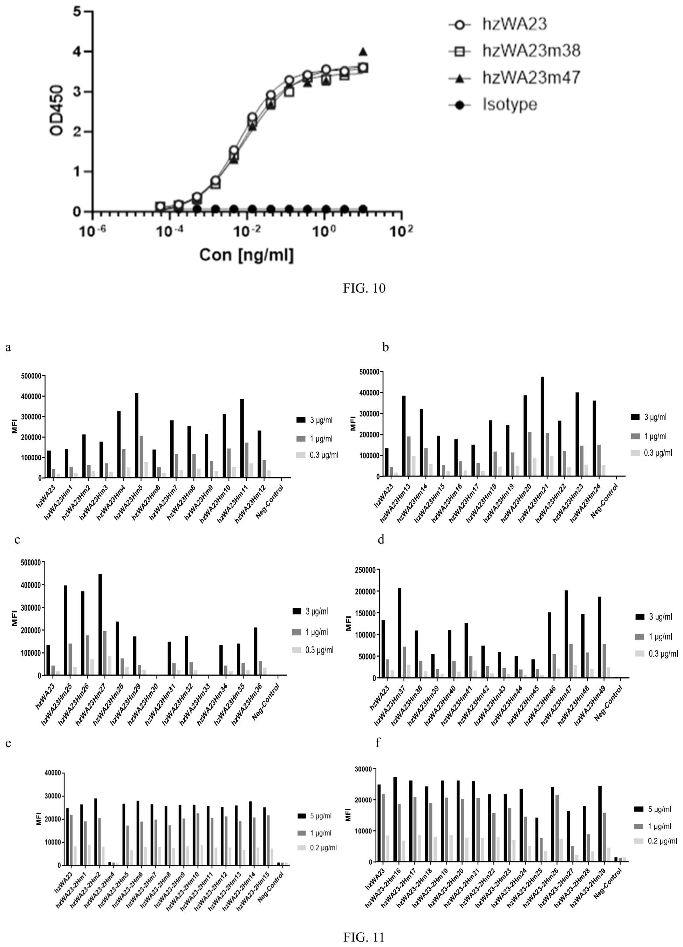

FIG. 10 is a graph showing the activity of hzWA23Hm38 and hzWA23Hm47 simultaneously binding to IL-18R1 and IL-18RAP by ELISA analysis in Example 14 according to the present application, where the concentrations of hzWA23Hm38 and hzWA23Hm47 are 10, 3.33, 1.11, 0.370, 0.123, 4.11E-2, 1.37E-2, 4.57E-3, 1.52E-3, 5.08E-4, 1.69E-4, and 5.65E-05 ng/ml.
FIG. 11 is a graph showing the binding activity of the hzWA23 mutant to IL-18R on the cell surface analyzed by FACS in Example 14 according to the present application, where a-d are graphs showing the binding activity of mutants on the hzR1-F9 side to the human IL-18R1 antigen on the cell surface at antibody concentrations of 3, 1, and 0.33 µg/ml; e and f are graphs showing the binding activity of mutants on the hzRAP-C10 side to the human IL-18RAP antigen on the cell surface at antibody concentrations of 5, 1, and 0. 2 µg/ml. The results showed that under the current experimental conditions, for most positions, the antibody mutants can bind to antigens on the cell membrane surface after histidine substitution.

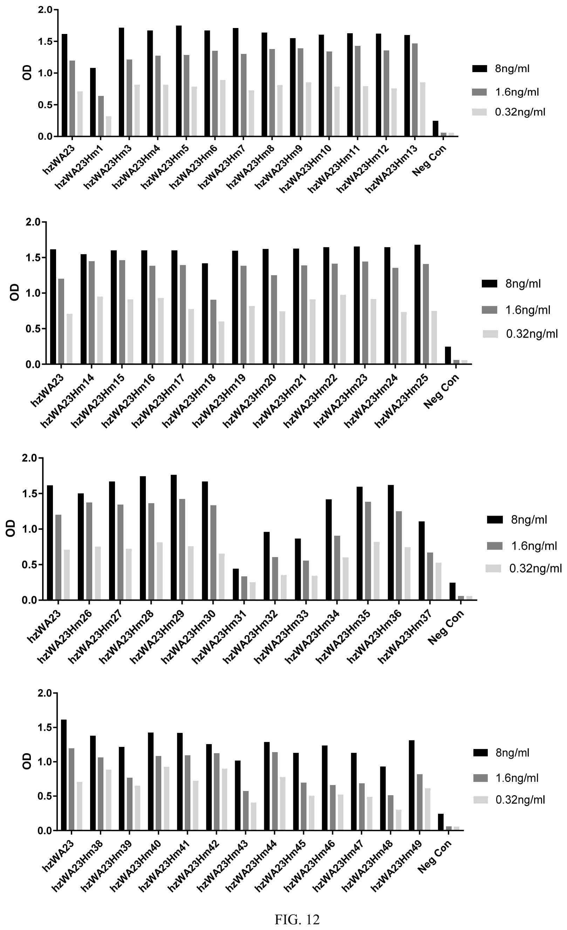

FIG. 12 is a graph showing the activation activity of the hzWA23 mutant analyzed by the reporter gene system in Example 15 according to the present application, where antibody concentrations are 8, 1.6, and 0.32 ng/ml. Under current experimental conditions, for most positions, the antibody can maintain good activation activity after histidine substitution.

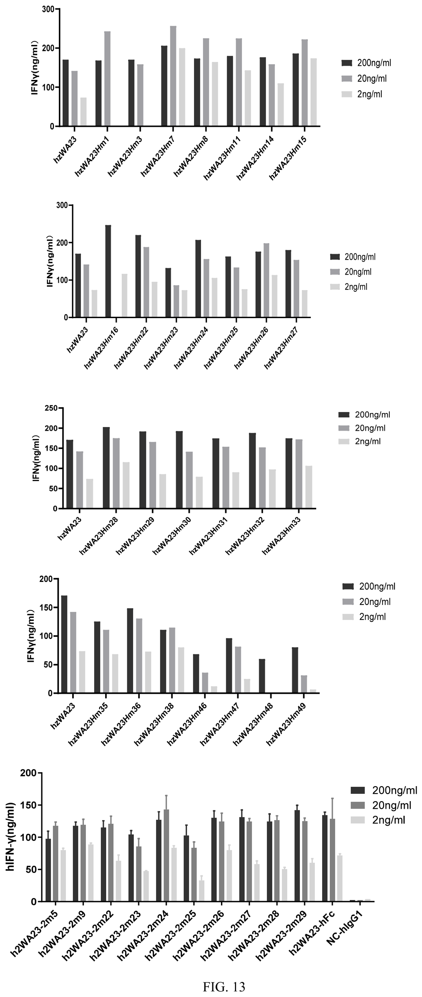

FIG. 13 is a graph showing the activation activity of the hzWA23 mutant analyzed by the PBMC activation experiment in Example 15 according to the present application, where antibody concentrations are 200, 20, and 2 ng/ml. Under current experimental conditions, for most positions, the antibody can maintain good activation activity for PBMCs after histidine substitution.

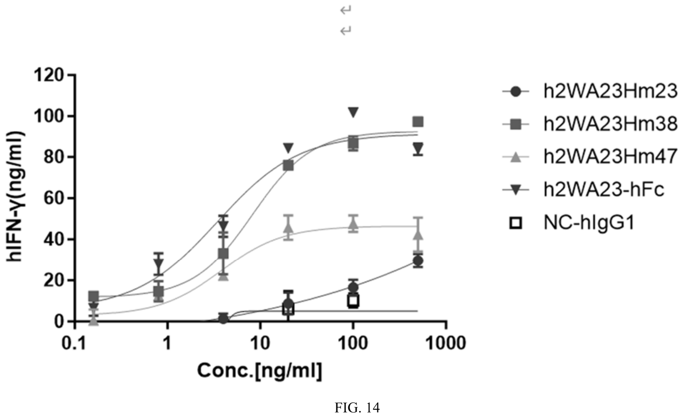

FIG. 14 is a graph showing the activation activity of the hzWA23 mutant analyzed by the CD3+T cell activation experiment in Example 15 according to the present application, where antibody concentrations are 500, 100, 20, 4, 0.8, and 0.16 ng/ml; The EC50 values of hzWA23Hm38 and hzWA23Hm47 for activating CD3+T cells are 8.25 ng/ml and 3.78 ng/ml, respectively, which are comparable to the activity of hzWAm23 (EC50=3.44 ng/ml), but the maximum activation intensity (Top value) of hzWA23Hm47 is lower. HzWAm23 has weak activity, and EC50 has not been calculated.

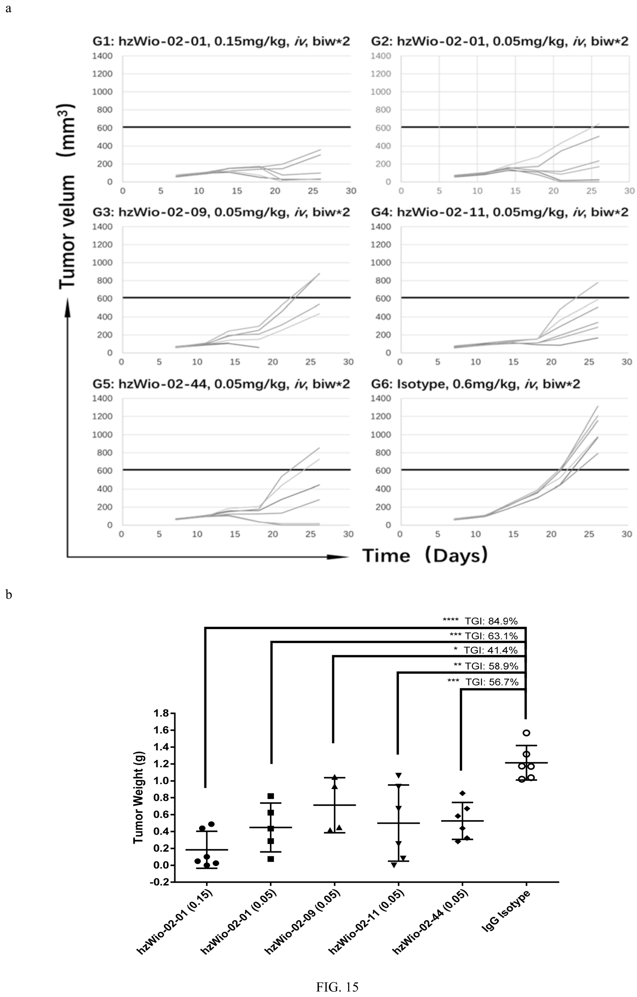

FIG. 15 is a graph showing the pharmacodynamic evaluation of hzWio-02-01, hzWio-02-09, hzWio-02-11, and hzWio-02-44 in the subcutaneous A375 cell-derived CDX model of the PBMC-reconstituted mice in Example 16 according to the present application, where a is a curve showing the tumor growth for the pharmacodynamic evaluation of hzWio-02-01, hzWio-02-09, hzWio-02-11, and hzWio-02-44 in the subcutaneous A375 cell line-derived CDX model of the PBMC-reconstituted mice, with each curve representing one mouse; and b is a graph showing the tumor weight for the pharmacodynamic evaluation of hzWio-02-01, hzWio-02-09, hzWio-02-11, and hzWio-02-44 in the subcutaneous A375 cell-derived CDX model of the PBMC-reconstituted mice..

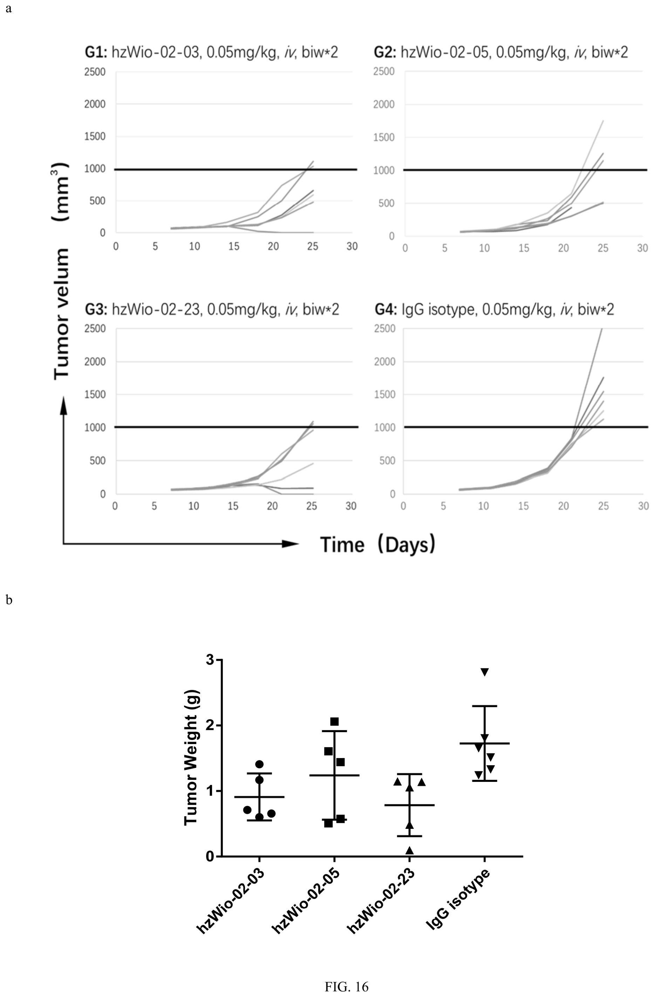

FIG. 16 is a graph showing the pharmacodynamic evaluation of hzWio-02-03, hzWio-02-05, and hzWio-02-23 in the subcutaneous A375 cell -derived CDX model of the PBMC-reconstituted mice in Example 16 according to the present application, where a is a curve showing the tumor growth for the pharmacodynamic evaluation of hzWio-02-03, hzWio-02-05, and hzWio-02-23 in the subcutaneous A375 cell -derived CDX model of the PBMC-reconstituted mice, with each curve representing one mouse; and b is a graph showing the tumor weight for the pharmacodynamic evaluation of hzWio-02-03, hzWio-02-05, and hzWio-02-23 in the subcutaneous A375 cell-derived CDX model of the PBMC-reconstituted mice.

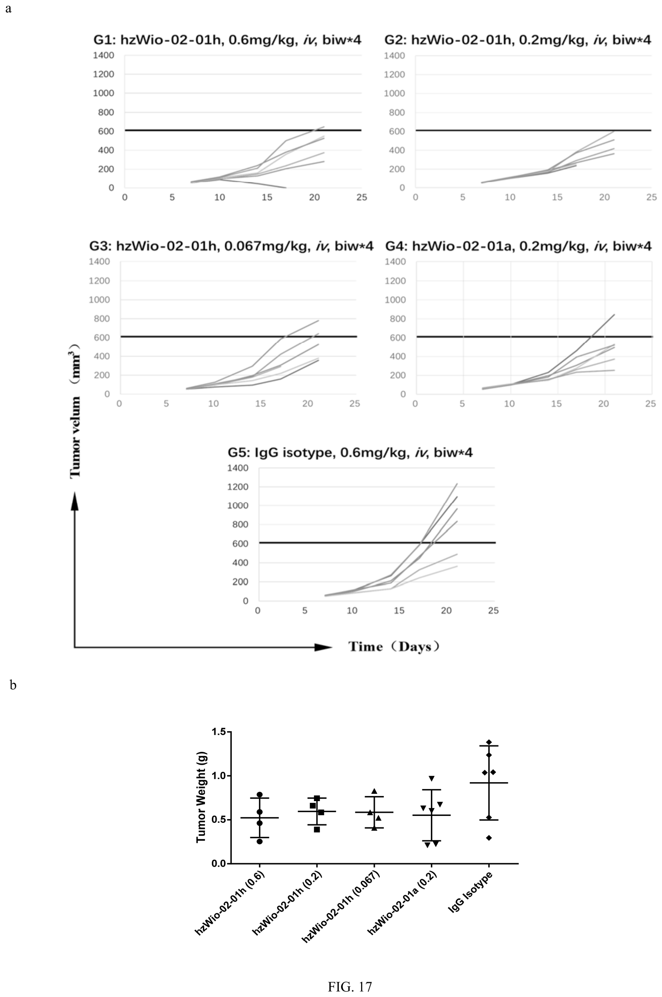

FIG. 17 is a graph showing the pharmacodynamic evaluation of hzWio-02-01a (G4) and hzWio-02-01h (G1, G2, G3) in the subcutaneous A375 cell-derived CDX model of the PBMC-reconstituted mice in Example 16 according to the present application, where a is a curve showing the tumor growth for the pharmacodynamic evaluation of hzWio-02-01a (G4) and hzWio-02-01h (G1, G2, G3) in the subcutaneous A375 cell -derived CDX model of the PBMC-reconstituted mice, with each curve representing one mouse; and b is a graph showing the tumor weight for the pharmacodynamic evaluation of hzWio-02-01a (G4) and hzWio-02-01h (G1, G2, G3) in the subcutaneous A375 cell-derived CDX model in the PBMC-reconstituted mice.

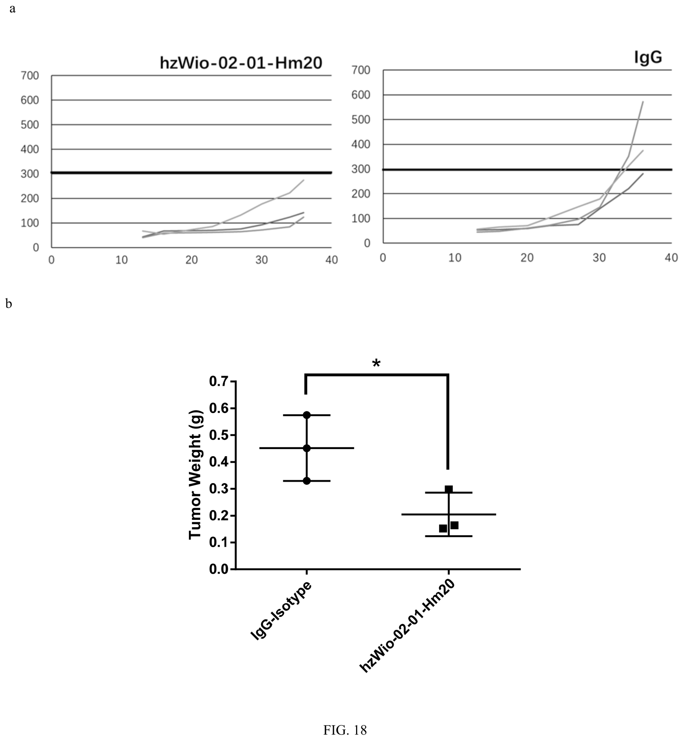

FIG. 18 is a graph showing the pharmacodynamic evaluation of hzWio-02-01-Hm20 in the subcutaneous HuCC-T1 cell-derived CDX model of the PBMC-reconstituted mice in Example 16 according to the present application, where a is a curve showing the tumor growth for the pharmacodynamic evaluation of hzWio-02-01-Hm20 in the subcutaneous HuCC-T1 cell-derived CDX model of the PBMC-reconstituted mice, with each curve representing one mouse; and b is a graph showing the tumor weight for the pharmacodynamic evaluation of hzWio-02-01 -Hm20 in the subcutaneous HuCC-T1 cell-derived CDX model of the PBMC-reconstituted mice.

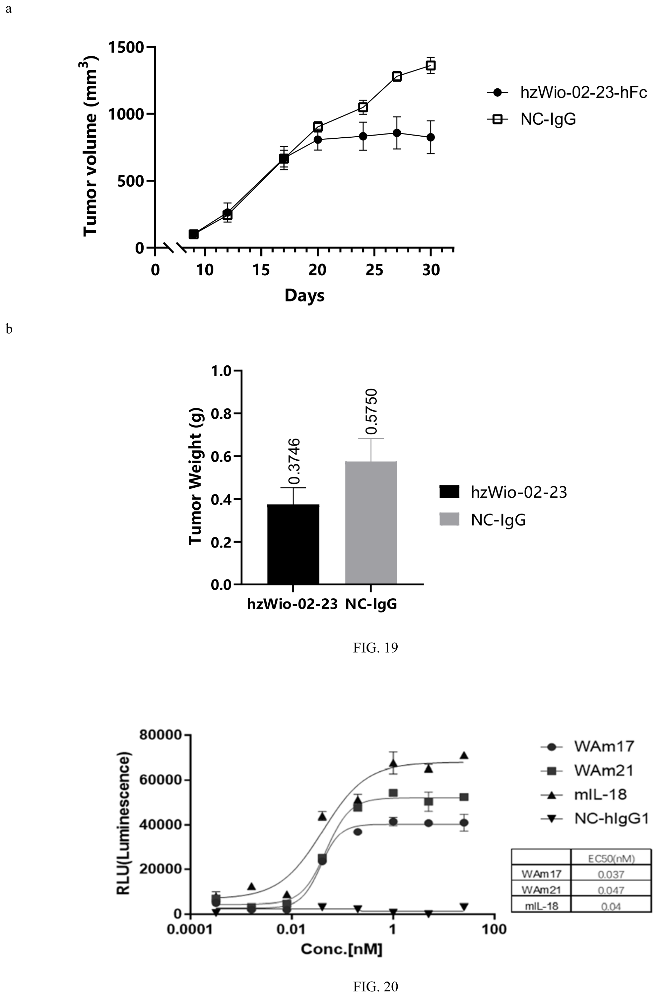

FIG. 19 is a graph showing the pharmacodynamic evaluation of hzWA23 in the CDX model with mixed inoculation of human tumor cells (human colorectal adenocarcinoma epithelial cells DLD-1) and human PBMCs in Example 16 according to the present application, where a is a curve showing the average tumor growth for the pharmacodynamic evaluation in the CDX model with mixed inoculation of human colorectal adenocarcinoma epithelial cells DLD-1 and human PBMCs, and b is a graph showing the tumor weight for the pharmacodynamic evaluation of hzWA23 in this model, with TGI = 34.9 %.
FIG. 20 is a graph showing the activation activity of WAm17 and WAm21 analyzed using Luciferase HEK 293 Cells in Example 18 according to the present application, where the working concentrations of WAm17 and WAm21 are 25, 5, 1, 0.2, 4.0E-2, 8.0E-3, 1.6E-3, and 3.2E-4 nM.

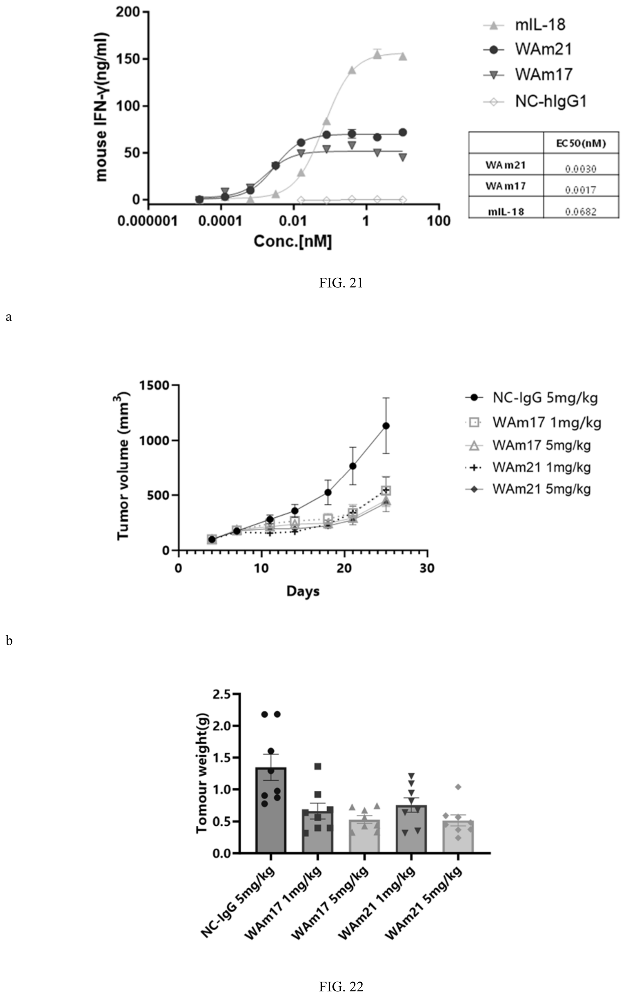

FIG. 21 shows the activity of WAm17 and WAm21 measured in the splenic immune cell activation assay in mice in Example 18 according to the present application, where the working concentrations are 10, 2, 0.4, 8.0E-2, 1.6E-2, 3.2E-3, 6.4E-4, 1.28E-4, and 2.56E-5 nM.
FIG. 22 is a graph showing the in vivo activity of agonistic camelid-derived anti-mouse IL-18R antibody evaluated in the mouse MC38 tumor model in Example 19 according to the present application, where a is a curve showing the tumor growth for the pharmacodynamic evaluation, and b is a graph showing the tumor weight for the pharmacodynamic evaluation.

### DETAILED DESCRIPTION

To clarify the purposes, solutions, and advantages of the present application, the solution in the present application will be described clearly and completely in conjunction with the accompanying drawings. It is obvious that the described embodiments are a part of the embodiments of the present application, not all of them. Based on the embodiments of the present application, all other embodiments obtained by those skilled in the art without creative labor are within the protection scope of the present application.

In the following Examples, camelid-derived single-domain antibodies targeting IL-18R1 or IL-18RAP are obtained through a camelid-derived single-domain immune antibody library. The single-domain antibodies of IL-18R1 and IL-18RAP are connected in tandem through a short peptide linker to obtain bispecific antibodies. Bispecific antibodies with IL-18-like biological functions that can activate the IL-18R1/IL-18RAP mediated intracellular signaling pathway are obtained through in vitro activity screening. The single-domain antibodies of anti-IL-18R1 and anti-IL-18RAP of bispecific antibodies were humanized, respectively, by humanization, and the humanized single-domain antibodies hz-aIL-18R1 and hz-aIL-18RAP were obtained, which were further combined into humanized bispecific antibodies. Cytological activity analysis was performed on the humanized bispecific antibodies to confirm their activity of activating the IL-18R1/IL-18RAP mediated signaling pathways and PBMCs. Through in vivo anti-tumor efficacy experiments, it is confirmed that bispecific antibodies have effective activity in inhibiting tumor.

### Example 1: The expression of recombinant proteins

### 1. Method for preparing Fc-tagged (or His-tagged) recombinant proteins

By using gene cloning, the coding gene of the target protein and the coding gene of the Fc-tag (or His-tag) were sequentially linked, and the resultant sequence was linked into the correct expression frame of a eukaryotic expression vector to construct a eukaryotic expression vector for a recombinant protein with the Fc-tag (or His-tag) fused at the C-terminus. After obtaining the expression vector plasmid with the correct sequence, HEK293 cells were transfected with the expression vector for transient expression. The expression supernatant was collected and purified using Protein A (or Ni-NTA) affinity chromatography column, and then desalinated into PBS by HiTrap desalination column. Finally, the recombinant protein with the Fc-tag (or His-tag) fused at the C-terminus was obtained. The type of the Fc tag described above includes a human-derived Fc tag (human Fc tag, hFc, SEQ ID NO.1), a mouse-derived Fc tag (mouse Fc tag, mFc, SEQ ID NO.2), and a camelid-derived Fc tag (Alpaca Fc tag, aFc, SEQ ID NO.3). The type of the His tag is 6 × His-tag (His, SEQ ID NO.4).

### 2. The preparation of IL-18R1/IL-18RAP recombinant protein

Referring to the above method, the gene encoding the N-terminal extracellular domain of human IL-18 receptor 1 (IL-18R1 ECD, SEQ ID NO. 5, NP_003846.1, Met1-Arg329) or the N-terminal extracellular domain of human IL-18-related receptor (IL-18RAP ECD, SEQ ID NO. 6; NP_003844.1, Met1-Gly357) was fused with hFc (SEQ ID NO. 1), mFc (SEQ ID NO. 2), aFc (SEQ ID NO. 3), or His-tag (SEQ ID NO. 4) for expression, and affinity chromatography column purification and desalination were performed to finally obtain recombinant proteins of IL-18R1 ECD-hFc (arranged sequentially from the N-terminus to the C-terminus as follows: the sequence set forth in SEQ ID NO. 5 and the sequence set forth in SEQ ID NO. 1), IL-18RAP ECD-mFc (arranged sequentially from the N-terminus to the C-terminus as follows: the sequence set forth in SEQ ID NO. 6 and the sequence set forth in SEQ ID NO. 2), IL-18R1 ECD-aFc (arranged sequentially from the N-terminus to the C-terminus as follows: the sequence set forth in SEQ ID NO. 5 and the sequence set forth in SEQ ID NO. 3), IL-18R1 ECD-His (arranged sequentially from the N-terminus to the C-terminus as follows: the sequence set forth in SEQ ID NO. 5 and the sequence set forth in SEQ ID NO. 4), and IL-18RAP ECD-hFc (arranged sequentially from the N-terminus to the C-terminus as follows: the sequence set forth in SEQ ID NO. 6 and the sequence set forth in SEQ ID NO. 1), IL-18R1 ECD-mFc (arranged sequentially from the N-terminus to the C-terminus as follows: the sequence set forth in SEQ ID NO. 5 and the sequence set forth in SEQ ID NO. 2), IL-18RAP ECD-aFc (arranged sequentially from the N-terminus to the C-terminus as follows: the sequence set forth in SEQ ID NO. 6 and the sequence set forth in SEQ ID NO. 3), and IL-18RAP ECD-His (arranged sequentially from the N-terminus to the C-terminus as follows: the sequence set forth in SEQ ID NO. 6 and the sequence set forth in SEQ ID NO. 4).

### Example 2: The screening of anti-human IL-18R1 or anti-human IL-18RAP camelid-derived heavy chain single-domain antibodies

IL-18R1 ECD-aFc (arranged sequentially from the N-terminus to the C-terminus as follows: the sequence set forth in SEQ ID NO. 5 and the sequence set forth in SEQ ID NO. 3) and IL-18RAP ECD-aFc (arranged sequentially from the N-terminus to the C-terminus as follows: the sequence set forth in SEQ ID NO. 6 and the sequence set forth in SEQ ID NO. 3) were used simultaneously as immunogens to immunize camels. Serum titers were measured by ELISA using IL-18R1 ECD-His (arranged sequentially from the N-terminus to the C-terminus as follows: the sequence set forth in SEQ ID NO. 5 and the sequence set forth in SEQ ID NO. 4) and IL-18RAP ECD-His (arranged sequentially from the N-terminus to the C-terminus as follows: the sequence set forth in SEQ ID NO. 6 and the sequence set forth in SEQ ID NO. 4) as detection antigens. After the serum titer met the requirements for library construction, peripheral blood mononuclear cells (PBMCs) were isolated, and total RNA was extracted for reverse transcription. The reverse transcription product was used as a template to amplify and isolate the variable domain of heavy chain of heavy-chain antibody (VHH), which was cloned into a phage display vector and electroporated into *Escherichia coli* TG1 competent cells to construct a 1.2×10⁹ camel anti-human IL-18R1/RAP immune antibody library. This antibody library was displayed to obtain a phage display library. The constructed camel immune library was screened three times by solid-phase screening using IL-18R1 ECD-His (arranged sequentially from the N-terminus to the C-terminus as follows: the sequence set forth in SEQ ID NO. 5 and the sequence set forth in SEQ ID NO. 4) and IL-18RAP ECD-His (arranged sequentially from the N-terminus to the C-terminus as follows: the sequence set forth in SEQ ID NO. 6 and the sequence set forth in SEQ ID NO. 4) as screening and identification antigens. Single clones were then selected and identified by ELISA after phage display (identification results are shown in Tables 1 and 2). Specific phage-displayed single-domain antibody positive clones against IL-18R1 or IL-18RAP were obtained, respectively. Phage-displayed plasmids (phagemids) were extracted from these monoclonal clones and reserved for future use.

**Table 1. The ELISA identification results of the anti-IL-18R1 phage antibody**

| Clone No. | 4-fold dilution | 16-fold dilution |
|---|---|---|
| R1-A2 | 0.892 | 0.588 |
| R1-A8 | 0.983 | 0.715 |
| R1-A12 | 2.707 | 2.637 |
| R1-C7 | 1.004 | 0.819 |
| R1-C10 | 1.331 | 0.977 |
| R1-C12 | 2.658 | 2.507 |
| R1-D2 | 1.113 | 0.717 |
| R1-D8 | 1.51 | 1.076 |
| R1-D10 | 2.58 | 2.56 |
| R1-E4 | 2.589 | 2.314 |
| R1-E7 | 2.847 | 2.883 |
| R1-E12 | 2.762 | 2.705 |
| R1-F9 | 2.036 | 1.631 |
| R1-F10 | 1.054 | 0.852 |
| R1-H9 | 1.253 | 0.955 |
| NC | 0.059 | 0.055 |

**Table 2. The ELISA identification results of the anti-IL-18RAP phage antibody**

| Clone No. | 4-fold dilution | 16-fold dilution |
|---|---|---|
| Rap-A3 | 2.194 | 1.882 |
| Rap-A10 | 1.826 | 1.566 |
| Rap-A11 | 1.735 | 1.418 |
| Rap-B1 | 1.808 | 1.312 |
| Rap-B8 | 1.857 | 1.669 |
| Rap-C6 | 1.979 | 1.776 |
| Rap-C10 | 1.852 | 1.577 |
| Rap-D7 | 2.714 | 2.079 |
| Rap-D10 | 2.112 | 1.8 |
| Rap-F11 | 1.998 | 1.584 |
| Rap-G4 | 2.339 | 2.047 |
| NC | 0.06 | 0.068 |

### Example 3: Construction of a miniature anti-human IL-18R1/RAP forward/reverse bispecific antibody library and clone selection

By PCR amplification, 15 gene fragments encoding anti-IL-18R1 single-domain antibodies and 11 gene fragments encoding anti-IL-18RAP single-domain antibodies were obtained and assembled. During assembly, the gene encoding the GGGGS linker was introduced between the two gene fragments using appropriately designed primers. The assembled gene fragments were then ligated into the pET32a vector (shown in FIG. 1, pET-Bis-R1/RAP) using genetic engineering methods such as enzyme digestion and ligation, and transformed into BL-21 competent cells to construct a miniature anti-human IL-18R1/RAP bispecific antibody library (forward library) with an amino acid sequence diversity of 165. By using the same method, but changing the connection order of the anti-IL-18R1 single-domain antibody and the anti-IL-18RAP single-domain antibody (shown in FIG. 1, pET-Bis-Rap/R1), a miniature anti-human IL-18RAP/R1 bispecific antibody library (reverse library) with an amino acid sequence diversity of 165 was constructed.

Monoclones were randomly selected from the above miniature anti-human IL-18R1/RAP (IL-18RAP/R1) bispecific antibody library and cultured overnight. They were transferred to 300 µl culture medium at a ratio of 1:10, cultured in a 96-well culture plate until OD reached 0.6-0.8, IPTG with a final concentration of 0.1 mM was added, followed by overnight induction at 30 °C. After centrifugation, the supernatant was separated and sterilized to obtain the soluble expression supernatant of bispecific antibody prokaryotic cells for future use.

### Example 4: The analysis of the binding activity of prokaryotically expressed anti-human IL-18R1/RAP bispecific antibody

ELISA plate was coated overnight with human IL-18R1 ECD-His protein (arranged sequentially from the N-terminus to the C-terminus as follows: the sequence set forth in SEQ ID NO. 5 and the sequence set forth in SEQ ID NO. 4) at 1 µg/ml and 50 µl/well. The ELISA plate was blocked with a blocking agent, and then the soluble expression supernatant diluted 25 times with the blocking agent was added. The plate was incubated at 37°C for 1 hour, washed with PBST, and IL-18RAP ECD-hFc (arranged sequentially from the N-terminus to the C-terminus as follows: the sequence set forth in SEQ ID NO. 5 and the sequence set forth in SEQ ID NO. 1) was added. The plate was incubated at 37°C for 30 minutes. Finally, HRP-labeled anti-human Fc secondary antibody (Cat: 115-035-071/lot: 144460) was added and incubated, and then the plate was washed, followed by color development. Clones exhibiting color development were considered capable of binding to both IL-18R1 and IL-18RAP simultaneously. 48 clones with high OD values were selected from both the forward and reverse libraries for further identification (see Tables 3 and 4 showing the results of the binding activity identification of the selected clones).

**Table 3. The results of the binding activity analysis of prokaryotically expressed anti-human IL-18R1/RAP forward bispecific antibodies**

| No. | IL-18-R1 ECD-his | | NC-his | |
|---|---|---|---|---|
| Wio-02-01 | 2.137 | 2.258 | 0.048 | 0.049 |
| Wio-02-02 | 1.777 | 1.786 | 0.046 | 0.047 |
| Wio-02-03 | 1.736 | 1.846 | 0.049 | 0.045 |
| Wio-02-04 | 1.754 | 1.721 | 0.047 | 0.046 |
| Wio-02-05 | 1.884 | 1.898 | 0.048 | 0.045 |
| Wio-02-06 | 2.004 | 1.978 | 0.047 | 0.051 |
| Wio-02-07 | 1.549 | 1.520 | 0.053 | 0.054 |
| Wio-02-08 | 1.796 | 1.914 | 0.055 | 0.050 |
| Wio-02-09 | 1.813 | 1.823 | 0.049 | 0.054 |
| Wio-02-10 | 1.588 | 1.608 | 0.052 | 0.051 |
| Wio-02-11 | 2.032 | 2.145 | 0.047 | 0.05 |
| Wio-02-12 | 1.982 | 1.99 | 0.046 | 0.046 |
| Wio-02-13 | 1.731 | 1.829 | 0.056 | 0.048 |
| Wio-02-14 | 1.780 | 1.898 | 0.055 | 0.043 |
| Wio-02-15 | 1.333 | 1.364 | 0.052 | 0.043 |
| Wio-02-16 | 1.097 | 1.154 | 0.054 | 0.055 |
| Wio-02-17 | 1.620 | 1.705 | 0.047 | 0.058 |
| Wio-02-18 | 1.275 | 1.338 | 0.055 | 0.042 |
| Wio-02-19 | 1.544 | 1.567 | 0.047 | 0.042 |
| Wio-02-20 | 1.970 | 1.998 | 0.045 | 0.042 |
| Wio-02-21 | 1.574 | 1.586 | 0.051 | 0.045 |
| Wio-02-23 | 1.731 | 1.829 | 0.056 | 0.048 |
| Wio-02-24 | 1.780 | 1.898 | 0.055 | 0.043 |
| Blank | 0.081 | 0.061 | 0.055 | 0.046 |

**Table 4. The results of the binding activity analysis of prokaryotically expressed anti-human IL-18R1/RAP reverse bispecific antibodies**

| No. | IL-18-R1 ECD-his | | NC-his | |
|---|---|---|---|---|
| Wio-02-25 | 1.268 | 0.961 | 0.045 | 0.176 |
| Wio-02-26 | 1.446 | 1.249 | 0.046 | 0.047 |
| Wio-02-27 | 1.269 | 2.032 | 0.046 | 0.045 |
| Wio-02-28 | 1.08 | 1.128 | 0.044 | 0.047 |
| Wio-02-29 | 2.046 | 2.041 | 0.044 | 0.045 |
| Wio-02-30 | 2.16 | 2.29 | 0.046 | 0.045 |
| Wio-02-31 | 2.171 | 2.184 | 0.047 | 0.044 |
| Wio-02-32 | 2.193 | 2.182 | 0.046 | 0.045 |
| Wio-02-33 | 1.537 | 1.403 | 0.045 | 0.051 |
| Wio-02-34 | 2.147 | 2.174 | 0.045 | 0.046 |
| Wio-02-35 | 2.384 | 2.358 | 0.049 | 0.046 |
| Wio-02-36 | 1.491 | 1.558 | 0.046 | 0.045 |
| Wio-02-37 | 1.395 | 1.448 | 0.048 | 0.050 |
| Wio-02-38 | 1.591 | 1.614 | 0.054 | 0.045 |
| Wio-02-39 | 1.274 | 1.319 | 0.047 | 0.043 |
| Wio-02-40 | 1.596 | 1.655 | 0.043 | 0.047 |
| Wio-02-41 | 1.193 | 1.191 | 0.058 | 0.041 |
| Wio-02-42 | 1.778 | 1.897 | 0.049 | 0.043 |
| Wio-02-43 | 1.586 | 1.600 | 0.040 | 0.050 |
| Wio-02-44 | 1.869 | 1.944 | 0.045 | 0.052 |
| Wio-02-45 | 1.766 | 1.835 | 0.046 | 0.052 |
| Wio-02-46 | 1.294 | 1.347 | 0.044 | 0.057 |
| Wio-02-47 | 1.885 | 1.949 | 0.060 | 0.042 |
| Wio-02-48 | 1.418 | 1.489 | 0.047 | 0.058 |
| Blank | 0.047 | 0.045 | 0.047 | 0.051 |

### Example 5: The activity evaluation of prokaryotically expressed anti-human IL-18R1/RAP bispecific antibody by reporter gene system

### 1. The reporter gene system of IL-18 Reporter HEK 293 Cells

IL-18 Reporter HEK 293 Cells were purchased from Beijing Kyinno Biotechnology (Beijing) Co., Ltd. (KC-2328). The reporter gene system was generated by expressing the NF - 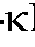 B/AP-1 inducible secretory embryonic alkaline phosphatase (SEAP) reporter gene in HEK293 cells, along with stable transfection of genes encoding human IL-18R1 and IL-18RAP. This system has a specific response to human IL-18. After binding to receptors on the cell surface, human IL-18 induced the expression of SEAP reporter genes downstream of the NF-κ B/AP-1 pathway, which catalyzed a chromogenic reaction with a specific substrate, indicating the activation of human IL-18. The activity testing results of recombinant human IL-18 (rhIL-18, Novoprotein, CH29) detected using this system is shown in FIG. 2, and the EC50 of rhIL-18 is 0.107 ± 0.020 ng/ml.

Similarly, this system could be used to detect the activation of the NF-κB/AP-1 pathway by agonistic bispecific antibodies targeting IL-18 receptors. When measuring activity, the cells were thawed, passaged, and cultured to a sufficient number, and collected and adjusted to an appropriate density. 180 µl of cell suspension (about 50,000 cells) and 20 µl of diluted sample to be tested (anti-human IL-18R1/RAP bispecific antibody) were added to each well of a 96-well plate, and a positive control (rhIL-18, Novoprotein, CH29) was set up. After mixing, the plate was incubated at 37°C in a CO₂ incubator for 20-24 hours. The next day, 180 µl of SEAP chromogenic substrate was added to each well of a new flat-bottom 96-well plate, followed by 20 µl of supernatant from induced HEK-Blue^{™} IL-18 cell. The plate was incubated at 37°C in an incubator for 1-3 hours, and the OD value at 630 nm was measured by a spectrophotometer. Resulting data were expressed as activation activity either by the OD value at a specific concentration (single concentration data) or by the EC50 (gradient concentration data). When using EC50, a variable slope curve of the concentration (log)-response (log(agonist) vs. Response, variable slope) was plotted, and then fitted with four-parameter non-linear regression curve to calculate EC50. The EC50 value was then combined with the maximum activation signal value (Top₆₃₀ₙₘ) to determine the activation activity of the tested sample.

### 2. Activation evaluation and sequence determination of prokaryotically expressed anti-human IL-18R1/RAP bispecific antibody

Referring to the above method, the reporter gene system of IL-18 Reporter HEK 293 Cells was used to detect the activity of prokaryotically expressed anti-human IL-18R1/RAP bispecific antibody in inducing the gene expression of the NF-κB/AP-1 pathway. The results are shown in Table 5.

**Table 5. The activation activity results of prokaryotically expressed anti-human IL-18R1/RAP bispecific antibody detected by the reporter gene system**

| Forward bispecific antibody | | | | Reverse bispecific antibody | | |
|---|---|---|---|---|---|---|
| Sample (supernatant ) | Dilution fold | | | Sample (supernatant ) | Dilution fold | |
| | 5X | 25X | | | 5X | 25X |
| Wio-02-01 | 2.131 | 1.915 | | Wio-02-25 | 0.623 | 0.268 |
| Wio-02-02 | 1.894 | 1.932 | | Wio-02-26 | 0.855 | 0.631 |
| Wio-02-03 | 1.385 | 0.608 | | Wio-02-27 | 0.686 | 0.263 |
| Wio-02-04 | 1.892 | 1.923 | | Wio-02-28 | 0.528 | 0.224 |
| Wio-02-05 | 1.967 | 1.863 | | Wio-02-29 | 1.510 | 1.233 |
| Wio-02-06 | 2.457 | 2.046 | | Wio-02-30 | 1.588 | 1.176 |
| Wio-02-07 | 0.606 | 0.449 | | Wio-02-31 | 1.655 | 1.771 |
| Wio-02-08 | 0.726 | 0.473 | | Wio-02-32 | 0.888 | 0.551 |
| Wio-02-09 | 1.911 | 1.874 | | Wio-02-33 | 0.535 | 0.286 |
| Wio-02-10 | 0.62 | 0.226 | | Wio-02-34 | 0.607 | 0.201 |
| Wio-02-11 | 2.85 | 2.17 | | Wio-02-35 | 0.678 | 0.262 |
| Wio-02-12 | 2.008 | 1.879 | | Wio-02-36 | 1.508 | 1.47 |
| Wio-02-13 | 1.688 | 1.262 | | Wio-02-37 | 1.478 | 1.518 |
| Wio-02-14 | 0.734 | 0.431 | | Wio-02-38 | 0.568 | 0.243 |
| Wio-02-15 | 0.577 | 0.206 | | Wio-02-39 | 0.656 | 0.246 |
| Wio-02-16 | 0.586 | 0.293 | | Wio-02-40 | 0.586 | 0.44 |
| Wio-02-17 | 1.639 | 1.248 | | Wio-02-41 | 1.514 | 1.233 |
| Wio-02-18 | 1.327 | 0.704 | | Wio-02-42 | 0.642 | 0.246 |
| Wio-02-19 | 0.927 | 0.859 | | Wio-02-43 | 0.682 | 0.267 |
| Wio-02-20 | 0.935 | 0.647 | | Wio-02-44 | 1.306 | 1.371 |
| Wio-02-21 | 1.796 | 1.799 | | Wio-02-45 | 1.087 | 0.427 |
| Wio-02-22 | 0.774 | 0.478 | | Wio-02-46 | 0.543 | 0.642 |
| Wio-02-23 | 1.778 | 1.903 | | Wio-02-47 | 1.667 | 1.251 |
| Wio-02-24 | 1.858 | 1.490 | | Wio-02-48 | 0.63 | 0.278 |
| NC | 0.0714 | 0.0677 | | NC | 0.0682 | 0.0663 |

Clones with intensive activation activity were selected for sequencing and comparative analysis, and duplicate sequences were removed. The results showed that the single-domain antibodies targeting the IL-18R1 side mainly include: R1-A12, R1-C7, R1-C10, R1-F9, and R1-E12 (sequences as shown in SEQ ID NOs.7-11, respectively). The single-domain antibodies targeting the IL-18RAP side include Rap-A10, Rap-C10, and Rap-F11 (sequences as shown in SEQ ID NOs.12-14, respectively). These sequences were combined with each other to form bispecific antibodies with a forward or reverse configuration and activation activity, the bispecific antibodies with a forward configuration and activation activity include:
Wio-02-01 (R1-A12-GGGGS-Rap-A10, SEQ ID NO.63);
Wio-02-03 (R1-C7-GGGGS -Rap-F11, SEQ ID NO.64);
Wio-02-05 (R1-C7-GGGGS - Rap-C10);
Wio-02-09 (R1-C10-GGGGS -Rap-F11);
Wio-02-11 (R1-C10-GGGGS -Rap-C10); and
Wio-02-23 (R1-F9-GGGGS - Rap-C10);
the bispecific antibodies with a reverse configuration and activation activity include:
   Wio-02-29 (Rap-A10-GGGGS-R1-A12);
   Wio-02-30 (Rap-C10-GGGGS-R1-C10);
   Wio-02-31 (Rap-C10-GGGGS-R1-F9); and
   Wio-02-44 (Rap-A10-GGGGS-R1-E12).

### Example 6: The preparation of Wio-02-01-hFc fusion protein and study on the effect of the linker with different lengths and sequences on activity

Referring to the method of constructing hFc fusion protein in Example 1, the bispecific antibody Wio-02-01 was fused with hFc (SEQ ID NO.1) and expressed. The anti-human IL-18R1/IL-18RAP bispecific antibody-hFc fusion protein, i.e., Wio-02-01-hFc (SEQ ID NO.65), was obtained by affinity chromatography and desalination. The linker between the single-domain antibody R1-A12 and the single-domain antibody Rap-A10 in this fusion protein is GGGGS (namely L0, SEQ ID NO.15).

The linker between the single-domain antibody R1-A12 and the single-domain antibody Rap-A10 in Wio-02-01-hFc was replaced with the following linker sequences: L1:GS; L2: GGGGSGGGGS (SEQ ID NO.17); L3: GGGGSGGGGSGGGGS (SEQ ID NO.18); and L4: GGGGSGGGGSGGGGSGGGGS (SEQ ID NO.19). Then, referring to the method of constructing hFc fusion protein in Example 1, bispecific antibodies with replaced linkers fused to hFc (SEQ ID NO: 1) were prepared, namely Wio-02-01-L1-hFe, Wio-02-01-L2-hFe, Wio-02-01-L3-hFc, and Wio-02-01-L4-hFc.

Finally, referring to the method described in Example 5, the in vitro activity of the anti-human IL-18R1/RAP bispecific antibody in the above form was evaluated using the reporter gene system, and the results are shown in FIG. 3. The result suggests that when the length of the linker increases to (G4S) 3 and (G4S)4, the activation activity of the bispecific antibody decreases, but it still maintains good activation activity. While other types of linkers have almost no impact on the activation activity of the bispecific antibodies. The above results indicate that anti-human IL-18R1/RAP bispecific antibodies can maintain good activation activity when GS flexible linkers of different lengths are used.

On the other hand, the linker (L0: GGGGS (SEQ ID NO: 15)) of the bispecific antibody hzWio-02-23 was replaced with the following linker sequences:
L5: GGGGSG (SEQ ID NO.66);
L6: GGGSGG (SEQ ID NO.67);
L7: GGSGGG (SEQ ID NO.68);
L8: GSGGGG (SEQ ID NO.69);
L9: GGSGSG (SEQ ID NO.70); and
L10: GSGSGG (SEQ ID NO.71).

Then, referring to the method of constructing hFc fusion protein in Example 1, the following fusion proteins of the bispecific antibody with the replaced linker and hFc were prepared:
hzWio-02-23-hFc;
hzWio-02-23-L5-hFc (SEQ ID NO.72);
hzWio-02-23-L6-hFc;
hzWio-02-23-L7-hFc;
hzWio-02-23-L8-hFc;
hzWio-02-23-L9-hFc; and
hzWio-02-23-L10-hFc,
wherein hzWio-02-23-L5-hFc is the fusion protein obtained by replacing the linker in hzWio-02-23-hFc with L5, with the other fusion proteins named accordingly by analogy.

Finally, referring to the method described in Example 5, the in vitro activity of the anti-human IL-18R1/RAP bispecific antibody in the above form was evaluated using the reporter gene system, and the results are shown in Table 6. When the linker was selected to be any one of the six different amino acid combinations mentioned above, the anti-human IL-18R1/RAP bispecific antibody maintained good activation activity. The above results further demonstrate that anti-human IL-18R1/RAP bispecific antibodies can maintain good activation activity when different GS flexible linkers are used.

**Table 6. The activity results of humanized anti-IL-18R1/RAP bispecific antibody with different linkers (6 aa Linker) evaluated by the reporter gene system**

| Sample | Top₆₃₀ₙₘ | EC50 (ng/ml) |
|---|---|---|
| hzWio-02-23-L0-hFc | 1.372 | 1.960 |
| hzWio-02-23-L5-hFc | 1.427 | 1.887 |
| hzWio-02-23-L6-hFc | 1.404 | 1.924 |
| hzWio-02-23-L7-hFc | 1.351 | 1.864 |
| hzWio-02-23-L8-hFc | 1.444 | 1.893 |
| hzWio-02-23-L9-hFc | 1.503 | 1.919 |
| hzWio-02-23-L10 | 1.476 | 1.821 |

### Example 7: The preparation and activation activity analysis of anti-human IL-18R1/RAP bispecific antibodies with different structural forms

Bispecific antibodies with different structural forms were constructed based on R1-A12/Rap-A10 single-domain antibodies, and their activity was analyzed and verified. The antibody structure forms are shown in FIG. 4, with a total of 8 structural forms from a to h, where structure a is the parent form of Wio-02-01 (R1-A12-GGGGS-Rap-A10). Other structural forms were obtained by combining R1-A12/Rap-A10 in new structures through means of different connections and tag fusion.

Referring to the method described in Example 5, the in vitro activity of various forms of anti-human IL-18R1/RAP bispecific antibodies was evaluated using the reporter gene system, and the results are shown in Table 7. The result suggests that all derivative structures forms containing an A-B tandem configuration, such as a, c, d, g, h, and the like, exhibited activation activity, and the Fc segment did not affect the activation activity of bispecific antibodies. However, b, e, and f, which did not contain a tandem configuration, only exhibited weak activation activity, indicating that the A-B tandem configuration is a key factor affecting activation activity.

**Table 7. The activation activity results of anti-human IL-18R1/RAP bispecific antibodies with different structural forms**

| Sample name | Activity (reporter gene system) | |
|---|---|---|
| | Top₆₃₀ₙₘ | EC50(ng/ml) |
| Wio-02-01a (Wio-02-01) | 0.906 | 1.871 |
| Wio-02-01b | 0.244 | 10.137 |
| Wio-02-01c | 1.077 | 1.478 |
| Wio-02-01d | 1.158 | 3.354 |
| Wio-02-01e | 0.287 | 7.644 |
| Wio-02-01f | 0.399 | 5.301 |
| Wio-02-01g | 0.876 | 1.762 |
| Wio-02-01h | 0.958 | 4.457 |
| Human IL-18 | 2.435 | 0.117 |

### Example 8: Humanization and eukaryotic expression of anti-human IL-18R1 or anti-human IL-18RAP heavy chain single-domain antibodies

Humanization of single-domain antibodies specific for human IL-18R1 or IL-18RAP was performed. Firstly, a comprehensive analysis of the VHH sequence of camelid-derived antibodies was conducted to determine the antigen complementary determining regions (CDRs) that bind the antibody to the antigen and the framework regions that support the conserved three-dimensional conformation of the antibody. Subsequently, based on the homology alignment results, the closest human antibody sequence was selected as the basic template. Combined with blast results of the full sequence, CDR grafting was performed to achieve humanization of the variable region (VHH) of the nanobody in the framework region. Subsequently, site-directed mutagenesis was performed on some key amino acids to maintain affinity comparable to that of the camelid-derived antibodies. The humanized sequences of single-domain antibodies specific for human IL-18R1 (hzR1-A12, hzR1-C7, hzR1-C10, and hzR1-F9, with sequences set forth in SEQ ID NOs. 20-23, respectively) and single-domain antibodies specific for anti-human IL-18RAP (hzRap-A10, hzRap-C10, and hzRap-F11, with sequences as shown in SEQ ID NOs. 24-26, respectively) were finally obtained.

Referring to the method of constructing hFc fusion protein in Example 1, the variable region coding genes of the anti-human IL-18R1 single-domain antibody, before and after humanization, were fused with hFc (SEQ ID NO.1) through the GS linker, and expressed. The anti-human IL-18R1 single-domain antibody-hFc fusion protein was obtained by affinity chromatography and desalination. The structure of the fusion proteins is shown in FIG. 5.

### Example 9: The preparation of humanized anti-human IL-18R1/RAP bispecific antibody

The humanized anti-human IL-18R1 or IL-18RAP specific heavy chain single-domain antibodies obtained in Example 8 were linked according to the structural form of the camelid-derived bispecific antibody with activation activity obtained in Example 5, to construct a tandem humanized bispecific antibody.

The humanized bispecific antibodies with a forward configuration include:
hzWio-02-01 (hzR1-A12-GGGGS-hzRap-A10, SEQ ID NO.59);
hzWio-02-03 (hzR1-C7-GGGGS-hzRap-F11, SEQ ID NO.60);
hzWio-02-05 (hzR1-C7-GGGGS-hzRap-C10, SEQ ID NO.61);
hzWio-02-09 (hzR1-C10-GGGGS-hzRap-F11);
hzWio-02-11 (hzR1-C10-GGGGS-hzRap-C10); and
hzWio-02-23 (hzR1-F9-GGGGS-hzRap-C10, SEQ ID NO.62).

The humanized bispecific antibodies with a reverse configuration include:
hzWio-02-29 (hzRap-A10-GGGGS-hzR1-A12);
hzWio-02-30 (hzRap-C10-GGGGS-hzR1-C10);
hzWio-02-31 (hzRap-C10-GGGGS-hzR1-F9); and
hzWio-02-44 (hzRap-A10-GGGGS-R1-E12, where R1-E12 are camelid-derived sequences).

Then, referring to the method of constructing the hFc fusion protein in Example 1, the humanized bispecific antibody was fused with hFc (SEQ ID NO.1) and expressed. The humanized anti-human IL-18R1/IL-18RAP bispecific antibody-hFc fusion protein was obtained by affinity chromatography and desalination. The structures of the fusion proteins are shown in FIG. 6.

The names and sequences of the fusion proteins are as follows:
hzWio-02-01-hFc, SEQ ID NO.59-SEQ ID NO.1;
hzWio-02-03-hFc, SEQ ID NO.60-SEQ ID NO.1;
hzWio-02-05-hFc, SEQ ID NO.61-SEQ ID NO.1;
hzWio-02-09-hFc, SEQ ID NO.22-SEQ ID NO.26-SEQ ID NO.1;
hzWio-02-11-hFc, SEQ ID NO.22-SEQ ID NO.25-SEQ ID NO.1;
hzWio-02-23-hFc, SEQ ID NO.62-SEQ ID NO.1;
hzWio-02-29-hFc, SEQ ID NO.24-SEQ ID NO.20-SEQ ID NO.1;
hzWio-02-30-hFc, SEQ ID NO.25-SEQ ID NO.22-SEQ ID NO.1;
hzWio-02-31-hFc, SEQ ID NO.25-SEQ ID NO.23-SEQ ID NO.1; and
hzWio-02-44-hFc, SEQ ID NO.24-SEQ ID NO.11-SEQ ID NO.1.

### Example 10: Affinity determination of anti-human IL-18R1 or anti-human IL-18RAP heavy chain single-domain antibodies

An anti-human Fc segment capture antibody biosensor probe was used to measure the ability of the human IL-18R1 (or human IL-18RAP) specific heavy chain single-domain antibody (fused with human Fc at the C-terminus) to bind to recombinant antigens by capturing the antibody Fc segment using Octet^{®} R4 system from Fortebio. When measuring, the sample to be tested was diluted with PBS buffer to 5 µg/mL, and reacted with an AHC probes (Cat: 18-0015, PALL) for immobilization onto the chip surface for 180 seconds. Recombinant protein human IL-18R1 ECD-His (arranged sequentially from the N-terminus to the C-terminus as follows: the sequence set forth in SEQ ID NO. 5 and the sequence set forth in SEQ ID NO. 4 ) or IL-18RAP ECD-His (arranged sequentially from the N-terminus to the C-terminus as follows: the sequence set forth in SEQ ID NO. 6 and the sequence set forth in SEQ ID NO. 4 ) served as the mobile phase and reacted with the antibody immobilized on the chip surface. The concentration of the recombinant protein was 100 nM. The binding time for each antigen was 300 seconds, and the dissociation time was 300 seconds. The results showed (Table 8) that under the current experimental conditions, both the anti-human IL-18R1 and anti-human IL-18RAP antibodies had high affinity for their corresponding antigens. Only the affinity of R1-C10 showed a significant decrease after humanization, compared with that before humanization, and the affinity of the other clones remained unchanged.

**Table 8. The affinity measurement results of anti-human IL-18R1 (or anti-human IL-18RAP) single-domain antibodies (fused with human Fc at the C-terminus)**

| Sample name | KD value (M) | kon (1/Ms) | kdis (1/s) |
|---|---|---|---|
| R1-A12 | 2.82E-09 | 1.68E+05 | 4.74E-04 |
| hzR1-A12 | 2.00E-09 | 9.01E+04 | 1.80E-04 |
| R1-C7 | 2.56E-09 | 2.09E+05 | 5.37E-04 |
| hzR1-C7 | 2.53E-09 | 2.04E+05 | 5.15E-04 |
| R1-C10 | 2.81E-11 | 5.22E+06 | 1.47E-04 |
| hzR1-C10 | 1.45E-09 | 1.42E+05 | 2.06E-04 |
| R1-F9 | 1.1E-09 | 7.94E+04 | 8.73E-05 |
| hzR1-F9 | 3.74E-09 | 5.69E+04 | 2.13E-04 |
| Rap-A10 | 5.30E-10 | 1.30E+06 | 6.87E-04 |
| hzRap-A10 | 9.86E-10 | 5.11E+04 | 5.04E-05 |
| Rap-C10 | 1.73E-09 | 6.06E+05 | 1.05E-03 |
| hzRap-C10 | 6.24E-09 | 1.85E+05 | 1.16E-03 |
| Rap-F11 | 4.58E-09 | 1.54E+05 | 7.05E-04 |
| hzRap-F11 | 4.85E-09 | 2.39E+05 | 1.16E-03 |

### Example 11: histidine scanning mutagenesis of bispecific antibody hzWio-02-01

Histidine scanning was performed on the three CDR regions at the R1-A12 side of hzWio-02-01 using site-directed mutagenesis. Specifically, primers for site-directed mutagenesis were designed, and the hzWio-02-01 coding gene was used as a template to introduce site-directed mutagenesis into the original gene sequence through PCR. Then, mutant recombinant proteins were prepared by eukaryotic expression and affinity purification, and were named hzWio-02-01-Hm1 to hzWio-02-01-Hm35 in order, which were respectively obtained by preforming T27H, A28H, Y29H, D30H, N31H, A32H, M34H, G35H, S50H, I51H, L53H, R54H, D55H, R56H, T57H, Y58H, Y59H, A60H, D61H, S62H, V63H, K64H, G65H, S98H, N99H, I100H, N102H, A103H, L104H, N105H, L106H, Q107H, G108H, N109H, and T110H mutations on the basis of SEQ ID NO. 59, wherein the sequence of hzWio-02-01-Hm1 is set forth in SEQ ID NO. 16.

### Example 12: Activity analysis of humanized anti-human IL-18R1/RAP bispecific antibody

### 1. The analysis of binding activity to antigens on the cell surface by FACS

Eukaryotic transient expression vectors for IL-18R1 and IL-18RAP full-length genes (IL-18R1, NP_003846.1; IL-18RAP, NP_003844.1) were constructed and transfected into HEK293 cells, separately. After 48 hours of culturing, the cells were collected and adjusted to 2×10⁵ cells/sample/100µL. The humanized anti-human IL-18R1/RAP bispecific antibodies (hzWio-02-01-hFc, hzWio-02-03-hFc, hzWio-02-05-hFc, hzWio-02-09-hFc, hzWio-02-11-hFc, and hzWio-02-23-hFc) were diluted, added to the cells at the working concentration, mixed well, and incubated at 4°C for 60 minutes. Then centrifugation was performed, and the supernatant was discarded. Cell culture medium was added to resuspend the cells and the cells were washed, and centrifuged again. The supernatant was discarded. Afterwards, flow cytometry antibody (Goat Anti-human IgG1-FITC, Sigma, F9512) was added, mixed well, incubated at 4°C for 30 minutes, the resultant was centrifuged and the supernatant was discarded. Finally, the cells were resuspended in cell culture medium and analyzed by flow cytometry. The result data were plotted into a variable slope curve (log(agonist) vs. Response, Variable slop) of the concentration (log)-response, and fitted with four-parameter non-linear regression curve to calculate EC50. The EC50 value was then combined with the maximum activation signal value (Top₄₁₂ₙₘ) to determine the binding activity of the tested sample. The results showed (FIG. 7) that the tested bispecific antibodies exhibited good binding activity to antigens on the cell surface.

### 2. The activity evaluation of humanized anti-IL-18R1/RAP bispecific antibody using a reporter gene system

Referring to the method of Example 5, the activity of the activity of anti-human IL-18R1/RAP bispecific antibodies in inducing NF-κB/AP-1 pathway was detected using the reporter gene system of IL-18 Reporter HEK 293 Cells. The results showed (Table 9) that the tested humanized bispecific antibodies can initiate the gene expression of the NF-κB/AP-1 pathway at extremely low concentrations (ng). However, compared to IL-18, the bispecific antibodies exhibited milder activation activity in the reporter gene system. The activity of the His-tag fusion form with a tandem structure was superior to that of the Fc tag form with a bivalent structure.

**Table 9. The activity results of the humanized anti-human IL-18R1/RAP bispecific antibody evaluated by the reporter gene system**

| Sample name | Fc form | | His form | |
|---|---|---|---|---|
| | Top₆₃₀ₙₘ | EC50 (ng/ml) | Top₆₃₀ₙₘ | EC50 (ng/ml) |
| hzWio-02-01 | 1.464 | 1.418 | 1.934 | 0.417 |
| hzWio-02-03 | 2.041 | 1.135 | 1.819 | 0.394 |
| hzWio-02-05 | 1.302 | 1.093 | 1.722 | 0.201 |
| hzWio-02-09 | 2.096 | 2.178 | 1.882 | 0.668 |
| hzWio-02-11 | 1.55 | 0.175 | 1.576 | 0.101 |
| hzWio-02-23 | 1.551 | 2.204 | 1.947 | 0.130 |
| hzWio-02-29 | 1.238 | 1.547 | 1.614 | 0.503 |
| hzWio-02-30 | 0.451 | 4.431 | 0.837 | 2.744 |
| hzWio-02-31 | 0.390 | 4.150 | 0.779 | 2.581 |
| hzWio-02-44 | 0.301 | 4.693 | 0.912 | 2.660 |
| Human IL-18 | / | / | 2.269 | 0.121 |

The activation activity of the hzWio-02-01 mutant was detected using the same method, and the results are shown in Table 10. The results suggest that mutations of amino acids of single-domain antibodies on the anti-IL-18R1 side affect the binding activity of this side, and alter the activation activity of the bispecific antibodies to some extent. However, all mutants maintained good activation activity. The activation activity of hzWio-02-01-Hm20 was significantly improved.

**Table 10. The activation activity results of the histidine scanning of hzWio-02-01 mutants (His-tag fusion form)***

| Sample name | Top₅₃₀ₙₘ | EC50 (ng/ml) | Sample name | Top₅₃₀ₙₘ | EC50 (ng/ml) |
|---|---|---|---|---|---|
| hzWio-02-01 | 1.049 | 2.301 | hzWio-02-01-Hm19 | 0.738 | 0.840 |
| hzWio-02-01-Hm1 | 0.909 | 1.255 | hzWio-02-01-Hm20 | 2.746 | 0.582 |
| hzWio-02-01-Hm2 | 1.097 | 2.274 | hzWio-02-01-Hm21 | 1.118 | 2.117 |
| hzWio-02-01-Hm3 | 1.082 | 1.135 | hzWio-02-01-Hm22 | 0.773 | 0.182 |
| hzWio-02-01-Hm4 | 1.077 | 0.941 | hzWio-02-01-Hm23 | 0.794 | 0.238 |
| hzWio-02-01-Hm5 | 1.006 | 1.106 | hzWio-02-01-Hm24 | 0.774 | 1.974 |
| hzWio-02-01-Hm6 | 0.894 | 1.077 | hzWio-02-01-Hm25 | 0.738 | 0.040 |
| hzWio-02-01-Hm7 | 1.030 | 1.122 | hzWio-02-01-Hm26 | 0.764 | 0.301 |
| hzWio-02-01-Hm8 | 1.510 | 1.012 | hzWio-02-01-Hm27 | 0.773 | 0.433 |
| hzWio-02-01-Hm9 | 0.900 | 2.144 | hzWio-02-01-Hm28 | 0.821 | 0.420 |
| hzWio-02-01-Hm10 | 1.102 | 1.273 | hzWio-02-01-Hm29 | 1.055 | 0.670 |
| hzWio-02-01-Hm11 | 1.019 | 1.419 | hzWio-02-01-Hm29 | 1.232 | 1.486 |
| hzWio-02-01-Hm12 | 1.168 | 0.680 | hzWio-02-01-Hm30 | 0.349 | 28.690 |
| hzWio-02-01-Hm13 | 1.224 | 1.118 | hzWio-02-01-Hm31 | 1.053 | 7.749 |
| hzWio-02-01-Hm14 | 1.250 | 1.259 | hzWio-02-01-Hm32 | 1.091 | 10.950 |
| hzWio-02-01-Hm15 | 1.128 | 1.536 | hzWio-02-01-Hm33 | 1.096 | 19.530 |
| hzWio-02-01-Hm16 | 1.115 | 1.146 | hzWio-02-01-Hm34 | 1.002 | 12.880 |
| hzWio-02-01-Hm17 | 1.004 | 1.471 | hzWio-02-01-Hm35 | 1.184 | 6.991 |
| hzWio-02-01-Hm18 | 1.070 | 0.696 | hzWio-02-01-Hm36 | 0.204 | ~ 288.6 |

| | | | | | |
|---|---|---|---|---|---|
| Note: *, activity detected by the reporter gene system. | | | | | |

### 3. The activity of the humanized anti-IL-18R1/RAP bispecific antibody evaluated by a PBMC activation experiment

Primary cultured human peripheral blood mononuclear cells (PBMCs) were used to evaluate the in vitro activity of anti-human IL-18R1/RAP bispecific antibodies. Human PBMCs were extracted and cultured in RPMI-1640 medium containing FBS and recombinant human interleukin-2 (rhIL-2) for 24 hours. Cells were collected and adjusted to 3×10⁶ cells/ml using medium containing IL12 (or IL15). Cells were inoculated into a cell culture plate, and the samples to be tested were added. After 48 hours of cultivation, the supernatant was collected, and a human IFN-γ detection kit (Biolegend human IFN-γ Kit) was used to measure the content of IFN-γ in the supernatant. A curve of the concentration (log)-IFN-γ content was plotted to calculate the Top value and EC50 value.

As shown in FIG. 8, under the current experimental conditions, in the presence of IL12 (or IL15), the bispecific antibodies can exert the effect of activating PBMC to secrete IFN-γ similar to IL-18. The bispecific antibodies had activation activities regardless of whether they are fused with hFc or His-Tag. The activation activity of different bispecific antibodies was somewhat different. With the exception of hzWio-02-01-Hm20-hFc, whose activity was essentially comparable to that of IL-18, the Emax values of activation activities of other molecules were significantly lower than that of IL-18, but the onset concentrations were significantly lower than that of IL-18. The above results suggest that the activation of the IL-18R1/RAP signaling pathway by the bispecific antibodies has the characteristics of a lower onset concentration but milder activation activity compared to that of IL-18, and they are expected to have better safety potential and a wider medication window.

Under the current experimental conditions, bispecific antibodies such as hzWio-02-01-hFc, hzWio-02-03-hFc, hzWio-02-05-hFc, hzWio-02-11-hFc, hzWio-02-23-hFc, and the like have good effects on stimulating PBMC to produce IFNγ, and their EC50 were better than that of rhIL-18, while the Emax values were significantly lower than that of IL-18.

Under the current experimental conditions, bispecific antibodies such as hzWio-02-01-His, hzWio-02-03-His, hzWio-02-05-His, hzWio-02-11-His, hzWio-02-23-His and the like have good effects on stimulating PBMC to produce IFNγ, and their EC50 were better than that of rhIL-18, while the Emax values were are significantly lower than that of IL-18.

Under the current experimental conditions, bispecific antibodies such as hzWio-02-01-His, hzWio-02-03-His, hzWio-02-05-His, hzWio-02-11-His, hzWio-02-23-His and the like have good effects on stimulating PBMC to produce IFNγ, and their EC50 were better than that of rhIL-18, while the Emax values were are significantly lower than that of IL-18.

Under the current experimental conditions, the activities of mutants hzWio-02-01-Hm11-hFc and hzWio-02-01-Hm20-hFc are significantly better than the parent hzWio-02-01-hFc. When compared with rhIL-18, the EC50 of the mutants was better than that of rhIL18, and the Top value was increased to a level close to rhIL-18.

### Example 13: Histidine scanning mutagenesis of hzWio-02-23-L5-hFc

Histidine scanning was performed on the three CDR regions of each of the two single-domain antibodies (hzR1-F9 and hzRAP-C10) contained in hzWio-02-23-L5-hFc (SEQ ID NO.72, hereinafter abbreviated as hzWA23) using site-directed mutagenesis. Specifically, primers for site-directed mutagenesis were designed and the hzWA23 coding gene was used as a template to introduce site-directed mutagenesis into the original gene sequence through PCR. Some mutants incorporated histidine mutations at two positions simultaneously (FIG. 9). Then, mutant recombinant proteins were prepared by eukaryotic expression and affinity purification. The mutants on the hzR1-F9 side were named hzWA23Hm1 to hzWA23Hm49 in order, which were obtained respectively by performing D31H, M32H, G33H, T48H, 149H, S50H, S51H, D52H, A53H, S54H, T55H, Y56H, Y57H, A58H, D59H, S60H, V61H, K62H, G63H, D96H, S97H, F98H, T99H, G100H, L101H, Q102H, L103H, A104H, E105H, K107H, A108H, D109H, F110H, G111H, Y112H, V29H, D52H/D59H, D52H/E105H, D52H/K107H, D59H/T99H, D59H/E105H, D59H/K107H, D59H/D109H, T99H/K107H, E105H/D109H, D52H/Y112H, D59H/Y112H, L101H/Y112H, E105H/Y112H mutations on the hzR1-F9 side of hzWA23 (SEQ ID NO.72). The mutants on the hzRAP-C10 side were named hzWA23-2Hml to hzWA23-2Hm29 in order, which were obtained respectively by performing A161H, Y162H, M163H, G164H, S179H, I180H, S181H, S182H, T183H, E184H, G185H, S186H, T187H, T188H, Y189H, A190H, D191H, S192H, V193H, K194H, G195H, Y228H, Q229H, F230H, G231H, A232H, F233H, R234H, V235H mutations on the hzRAP-C10 side of hzWA23 (SEQ ID NO.72).

### Example 14: Analysis of binding activity of hzWA23 mutant

### 1. The analysis of binding activity to recombinant antigen by Fortebio

An anti-human Fc segment capture antibody biosensor probe was used to measure the ability of different mutants of hzWA23 to bind to recombinant antigens by capturing the antibody Fc segment using Octet^{®} R4 system from Fortebio. When measuring, the sample to be tested was diluted with PBS buffer to 5 µg/mL, and reacted with AHC probes (Cat: 18-0015, PALL) for immobilization onto the chip surface for 180 seconds. Recombinant protein human IL-18R1 ECD-His or IL-18RAP ECD-His served as the mobile phase and reacted with the antibody immobilized on the chip surface. The recombinant proteins were dissolved in phosphate buffer solutions with pH 7.4 and 6. 0, respectively, at concentrations of 60 nM, and then reacted with antibodies already coated on the chip surface. The binding time for each antigen was 300 seconds, and the dissociation time was 300 seconds. The results showed (Table 11, and Table 12) that under the current experimental conditions. For most positions, the mutants could maintain good affinity after histidine substitution. Under the condition of pH 6.0, most mutants have higher affinity.

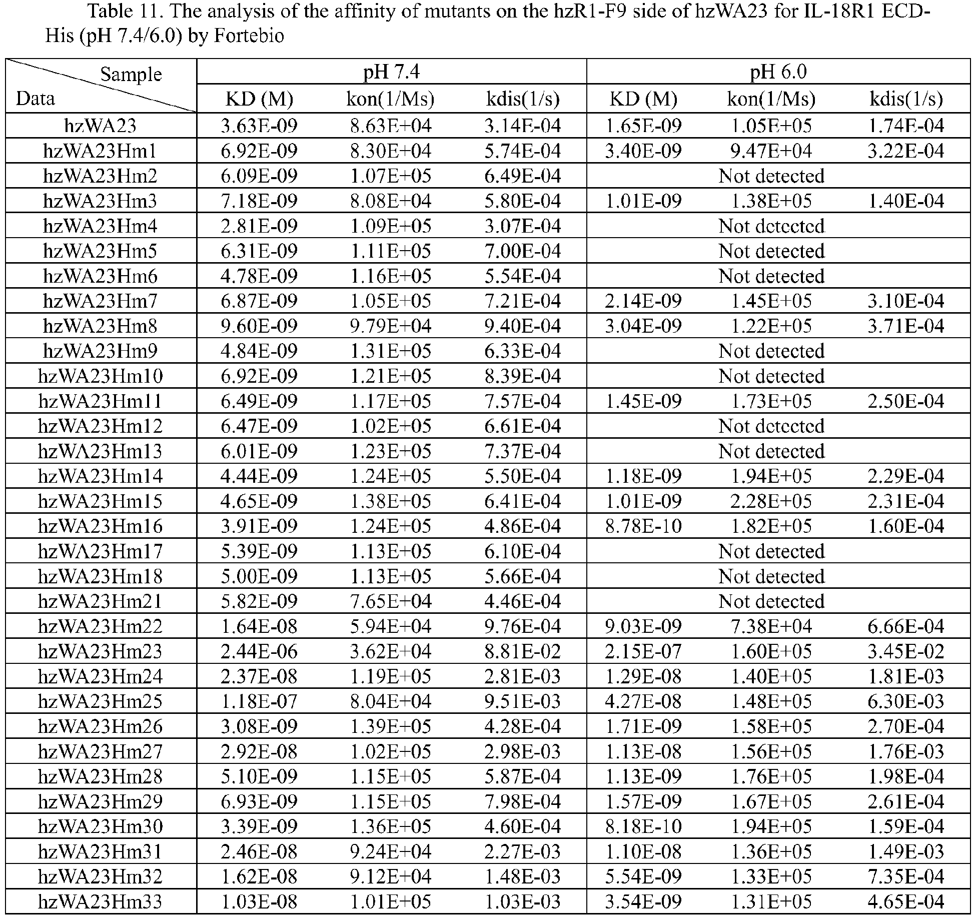

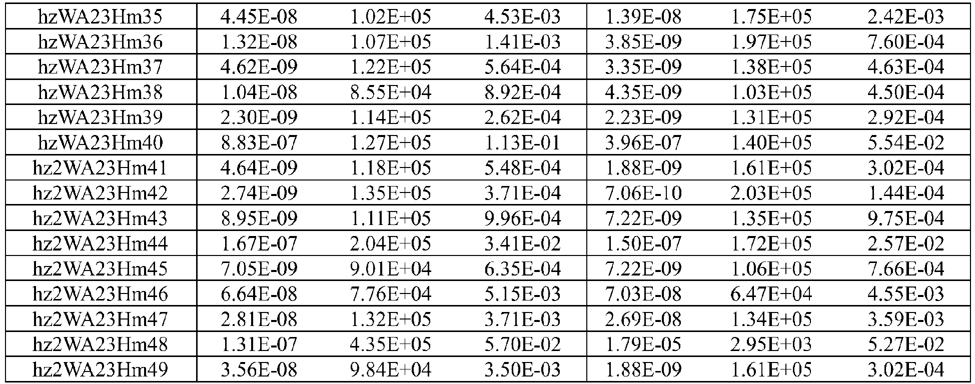

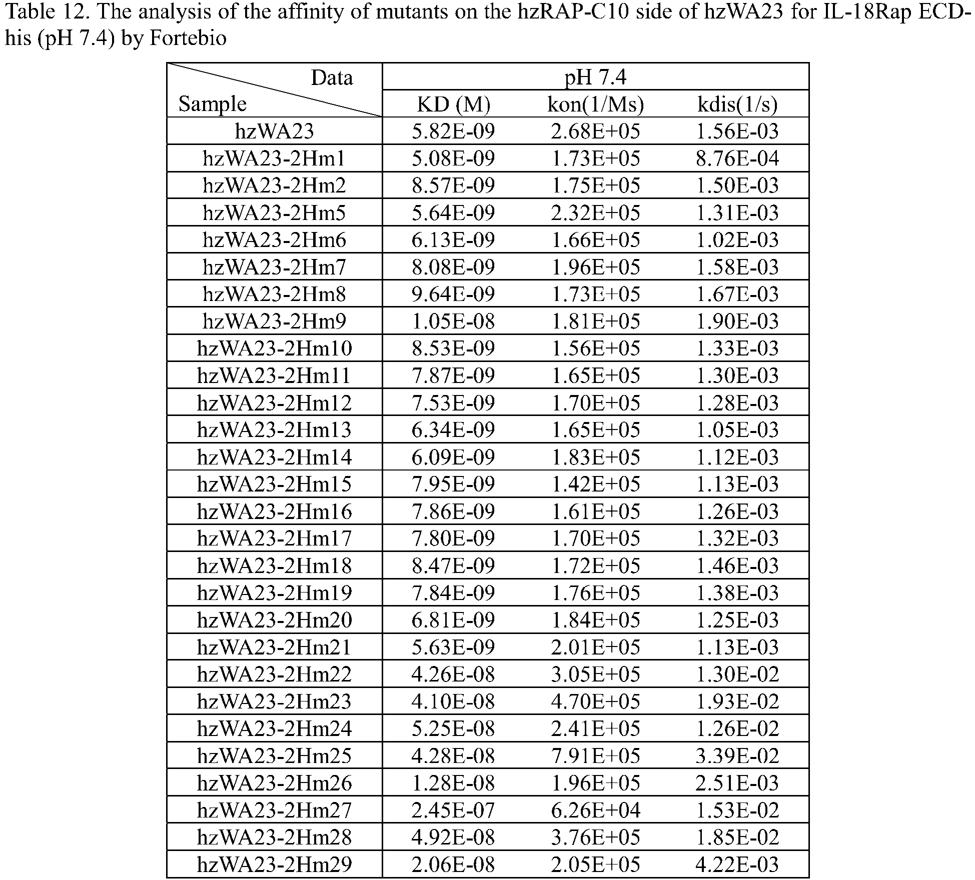

### 2. The analysis of the activity of hzWA23Hm38 and hzWA23Hm47 to simultaneously bind to IL-18R1 and IL-18RAP by ELISA

An ELISA plate was overnight coated with human IL-18R1 ECD-His protein at a concentration of 1 µg/ml and 50 µl/well. The ELISA plate was blocked with a blocking agent, and then diluted samples (hzWA23, hzWA23Hm38 (SEQ ID NO.73), hzWA23Hm47 (SEQ ID NO.74)) to be tested were added. The plate was incubated at 37°C for 1 hour, and washed with PBST. 1 µg/ml of IL-18RAP ECD-mFc was added, and incubated at 37°C for 30 minutes. Finally, HRP-labeled anti-mouse Fc secondary antibody was added and incubated, the plate was washed, followed by color development. A curve of OD-concentration was plotted. The results showed (FIG. 10) that hzWA23 and its mutants (hzWA23Hm38 and hzWA23Hm47) could simultaneously bind to IL-18R1 and IL-18RAP.

### 3. The analysis of binding activity to antigens on the cell surface by FACS

Eukaryotic transient expression vectors for IL-18R1 and IL-18RAP full-length genes (IL-18R1, NP_003846.1; IL-18RAP, NP_003844.1) were constructed separately and transfected into HEK293 cells. After 48 hours of culturing, the cells were collected and adjusted to 2×10⁵ cells/sample/100 µL. The hzWA23 and its mutants were diluted, added to the cells at the working concentration, mixed well, and incubated at 4°C for 60 minutes. Then centrifugation was performed, and the supernatant was discarded. Cell culture medium was added to resuspend the cells and the cells were washed, and centrifuged again to discard the supernatant. Afterwards, flow cytometry antibody (Goat Anti-human IgG1-FITC, Sigma, F9512) was added, mixed well, incubated at 4°C for 30 minutes, followed by centrifugation to discard the supernatant. Finally, the cells were resuspended with cell culture medium and analyzed by flow cytometry. The results showed (FIG. 11) that under the current experimental conditions, for most positions, hzWA23 mutants maintained good binding activity to antigens on the cell membrane surface after histidine substitution.

### Example 15: The analysis of the activation activity of the hzWA23 mutant

(1) The activity of hzWA23 mutants in inducing activation of the NF-κB/AP-1 pathway wad detected using the reporter gene system of IL-18 Reporter HEK 293 Cells, and the OD values resulting from the chromogenic substrate catalyzed by the reporter gene SEAP were detected. The results showed (FIG. 12) that most of the mutants maintained good activation activity, similar to the activation characteristics of the parent antibody.
(2) The in vitro activation activity of the mutants was assessed using primarily cultured human peripheral blood mononuclear cells (PBMCs) by measuring the IFN-γ content in the culture supernatant after treatment. The test results (FIG. 13) showed that most of the mutants maintained good activation activity, similar to the activation characteristics of the parent antibody.
(3) The in vitro activation activity of hzWA23Hm23 (SEQ ID NO.75), hzWA23Hm38, and hzWA23Hm47 was evaluated using T cells (CD3+) isolated from primary cultured human PBMCs. Human PBMCs were extracted, and T cells were isolated using CD3+ antibody beads. The obtained cells were cultured in RPMI-1640 medium containing FBS and recombinant human interleukin-2 (rhIL-2) for 24 hours. Cells were collected and adjusted to 2×10⁶ cells/ml using medium containing IL12. Cells were inoculated into a cell culture plate, and the samples to be tested were added. After 48 hours of cultivation, the supernatant was collected, and a human IFN-γ detection kit (Biolegend human IFN-γ Kit) was used to measure the content of IFN-γ in the supernatant. A curve of the concentration (log)-IFN-γ content was plotted to calculate the EC50 value. The results are shown in FIG. 14.

### Example 16: The evaluation of in vivo activity of humanized anti-human IL-18R1/RAP bispecific antibody

Human tumor cells (melanoma cell line A375) were inoculated subcutaneously into the right anterior rib of NSG mice reconstituted with human PBMCs. When the tumor grew to 60-100 mm³, the mice were divided into groups and administered with anti-human IL-18R1/RAP bispecific antibodies via tail vein injection. At the same time, an isotype IgG group was set as a control. During the experiment, tumor volume and weight were measured twice a week, and the relationship between changes in weight and tumor volume of tumor-bearing mice and the administration time was recorded. At the end of the experiment, the tumor-bearing mice were euthanized and their tumors were removed, weighed, and photographed. The relative tumor volume ratio (T/C) and tumor growth inhibition rate (1-T/C) between the treatment group and the control group were calculated and statistically analyzed. The experimental results are shown in FIGS. 15, 16, 17, and 18. This results indicate that under the current experimental conditions, multiple bispecific antibodies (hFc structure) such as hzWio-02-01, hzWio-02-03, hzWio-02-05, hzWio-02-09, hzWio-02-11, hzWio-02-23, and hzWio-02-44 with a reverse structure all exhibited clear tumor-inhibiting effects with a dose-response relationship (FIG. 15, and FIG. 16). The hzWio-02-01a without Fc structure and the hzWio-02-01h with a complex structure also have clear tumor-inhibiting effects (FIG. 17). The highly active mutant hzWio-02-01-m20 of hzWio-02-01 was selected and evaluated in a cell tumor model of human cholangiocarcinoma cell (HuCC-T1). The results also showed clear activity to inhibit tumor growth (FIG. 18). The results suggested that the bispecific antibodies with different structural forms possessing cytological agonistic activity have the potential to be developed as anti-tumor drugs.

Human tumor cells (human colorectal adenocarcinoma epithelial cells DLD-1) and human PBMCs were mixed in a ratio of 10:1 and inoculated subcutaneously into the right anterior rib of NSG mice. When the tumor grew to 60-100 mm³, the mice were divided into groups and administered with hzWA23 via tail vein injection. At the same time, an isotype IgG group was set as a control. There were 6 mice in each group, and the administration dosage was 0.3 mg/kg, with two administrations performed on Day 1 and Day 4 after grouping. During the experiment, tumor volume was measured twice a week, and the relationship between changes in tumor volume of tumor-bearing mice and administration time was recorded. At the end of the experiment, the tumor-bearing mice were euthanized, and their tumors were removed and weighed. The relative tumor volume ratio (T/C) and tumor growth inhibition rate (TGI = 1-T/C) between the treatment group and the control group were calculated and statistically analyzed. The experimental results are shown in FIG. 19. The results indicated that under the current experimental conditions, hzWA23 exhibited a clear tumor-inhibiting effect, with TGI =34. 9%.

### Example 17: The preparation of activated camelid-derived anti-mouse IL-18R antibody

To further demonstrate that the structural forms of anti-IL-18R1 and IL-18Rap bispecific antibodies have universal in vitro and in vivo activity, an alternative molecule of hzWA23 that binds to mouse IL-18R1 and IL-18Rap was constructed with reference to the preparation protocol of hzWA23. Specifically, the fusion protein of the N-terminal extracellular domain of recombinant mouse IL-18 receptor 1 (mIL-18R1 ECD, NCBI: NP_032391.1) and alpaca Fc (aFc), and the fusion protein of the N-terminal extracellular domain of recombinant mouse IL-18 related receptor (IL-18RAP ECD, NCBI: NP_034683.1) and alpaca Fc (aFc), were used as immunogens, respectively. After mixing the antigen with Freund's adjuvant in a volume ratio of 1:1 and emulsifying thoroughly, adult camels aged 3-5 years old were selected and immunized for subcutaneous injection at a dose of 1 mg/injection/animal. The first immunization used Freund's complete adjuvant, followed by five subsequent immunizations with Freund's incomplete adjuvant, administered every 2 weeks. Ten days after the fifth immunization, camel peripheral blood was collected, and mononuclear cells (PBMCs) were isolated. RNA was extracted and reverse transcribed to obtain a gene fragment library for variable domains of single-domain antibodies (VHH). Subsequently, a VHH phage antibody library was constructed based on this, and single-domain antibodies specific to mouse IL-18R1 and mouse IL-18RAP were screened and obtained. By combining single-domain antibodies of mouse IL-18R1 and mouse IL-18RAP, anti-mIL-18R1/mIL-18Rap-hFc fusion proteins (WAm17 and WAm21) with the same molecular structure as hzWA23 were constructed.

### Example 18: The evaluation of the in vitro activity of agonistic camelid-derived anti-mouse IL-18R antibody

(1) The activation activity of WAm17 and WAm21 was analyzed using Luciferase HEK 293 Cells. Luciferase HEK 293 Cells expressed the NF-κB/AP-1 induced luciferase reporter gene. After transient transfection of genes encoding mouse IL-18R1 and IL-18RAP, the IL-18R1 and IL-18RAP were expressed on the cell membrane, followed by binding to mouse IL-18, which mediated the expression of luciferase reporter genes downstream of the NF-κB/AP-1 pathway and catalyzed a chromogenic reaction with specific substrates, indicating the activation of mouse IL-18. Similarly, this system can be used to detect the activation of the NF-κB/AP-1 pathway by anti-mouse IL-18R antibodies. The activation activity of WAm17 and WAm21 was measured using this system. When measuring the activity, the cells were thawed, passaged, and cultured to a sufficient number, collected and adjusted to an appropriate density, and transfected with mouse IL-18R1 and IL-18RAP transient expression vectors. After 24 hours of transfection, cells were collected and adjusted to an appropriate density. 80 µl of cell suspension (about 20,000 cells) and 20 µl of diluted sample to be tested (WAm17, WAm21, mIL-18) were added to each well of a 96-well plate. After mixing, the plate was incubated at 37°C in a CO₂ incubator for 20-24 hours. The next day, 100 µl of luciferase chromogenic substrate was added to each well of the culture plate. After sufficient cell lysis and reaction, the luminescent signal value was measured using a spectrophotometer. The result data were plotted into a variable slope curve (log(agonist) vs. response, Variable slope) of the concentration (log)-response, and then fitted with four-parameter non-linear regression curve to calculate EC50. The measurement results showed that both WAm17 and WAm21 could activate the expression of reporter genes in the downstream signaling pathway of mouse IL-18 (FIG. 20).
(2) Primarily cultured immune cells isolated from the mouse spleen were used to analyze the activation activity of WAm17 and WAm21. Immune cells from mouse spleen were extracted and cultured in RPMI-1640 medium containing FBS and recombinant mouse interleukin-2 (rhIL-2) for 24 hours. Cells were collected and adjusted to 2×10⁶ cells/ml using medium containing mouse IL12. Cells were inoculated into a cell culture plate, and the samples to be tested were added. After 48 hours of cultivation, the supernatant was collected, and a mouse IFN-γ detection kit (ELISA MAX^{™} Standard Set Mouse IFN-γ Cat. 430801, Biolegend) was used to measure the content of IFN-γ in the supernatant. As shown in FIG. 21, both WAm21 and WAm17 can dose-dependently stimulate the secretion of cytotoxic factor IFN-γ by mouse immune cells, and their EC50 values were better than that of mouse IL-18 (abbreviated as mIL-18). The maximum activation ability caused by WAm21 and WAm17 was weaker than that of mIL-18. The results indicate that the mechanisms and characteristics of WAm21 and WAm17 in activating mouse immune cells were similar to those of hzWA23, and WAm21 and WAm17 could be used as alternative molecules of hzWA23 for in vivo efficacy evaluation of mIL-18 activators.

### Example 19: The evaluation of in vivo activity of agonistic camelid-derived anti-mouse IL-18R antibody

Mouse tumor cells (melanoma cell line MC38) were inoculated subcutaneously into the right anterior rib of the mouse. When the tumor grew to 60-100 mm³, the mice were divided into groups and administered with anti-mouse IL-18R agonistic antibodies (WAm21 and WAm17) via the intraperitoneal injection. At the same time, an isotype IgG group was set as a control. The grouping information is shown in Table 13. During the experiment, tumor volume and weight were measured twice a week, and the relationship between changes in weight and tumor volume of tumor-bearing mice and administration time was recorded. At the end of the experiment, the tumor-bearing mice were euthanized and their tumors were removed, weighed, and photographed. The tumor growth and tumor weight inhibition rate were calculated and statistically analyzed. The experimental results are shown in FIG. 22 and Table 14. The experimental results indicated that under the current experimental conditions, WAm17 and WAm21 exhibited clear activity to inhibit tumor growth.

**Table 13. The grouping information table for evaluating the efficacy of WAm17 and WAm21 in the mouse colorectal cancer MC38 CDX tumor model**

| Group (n=6) | Test substance | Dosage (mg/kg) | Administration route | Administration time |
|---|---|---|---|---|
| G1 | NC | 5 | i.p | QW× 4 |
| G2 | WAm17 | 1 | i.p | QW× 4 |
| G3 | WAm17 | 5 | i.p | QW× 4 |
| G4 | WAm21 | 1 | i.p | QW× 4 |
| G5 | WAm21 | 5 | i.p | QW× 4 |

**Table 14. The efficacy results of WAm17 and WAm21 in the mouse colorectal cancer MC38 CDX tumor model**

| Group | Tumor volume (TV, D25) | | Tumor weight (TW) | |
|---|---|---|---|---|
| | TGI (%) | P (T.test) | TGI (%) | P (T.test) |
| NC 5mg/kg | / | / | / | / |
| WAm17 1mg/kg | 54.8% | 0.040 | 49.2% | 0.012 |
| WAm17 5mg/kg | 57.7% | 0.017 | 59.3% | 0.002 |
| WAm21 1mg/kg | 51.0% | 0.041 | 42.0% | 0.024 |
| WAm21 5mg/kg | 60.0% | 0.015 | 60.4% | 0.002 |

Finally, it should be noted that the above embodiments are only used to illustrate the solutions of the present application, but not to limit it. Although the present application has been described in detail with reference to the aforementioned embodiments, those skilled in the art should understand that they can still modify the solutions described in the aforementioned embodiments, or equivalently, replace some of the features. However, these modifications or replacements do not deviate the essence of the corresponding solutions from the spirit and scope of the solutions of the embodiments of the present application.

### Industrial Applicability

There is provided an antibody that specifically binds to the IL-18 receptor and an antibody that specifically binds to IL-18RAP, which have high affinity for IL-18R1 and IL-18RAP according to the present application. The bispecific antibodies provided by the present application can efficiently bind to IL-18R1 and IL-18RAP simultaneously, activate the IL-18 signaling pathway, and exert IL-18-like biological functions both in vitro and in vivo. As a novel form of IL-18 receptor activator, it has a relatively broad-spectrum immune enhancement effect, stimulating the proliferation and activation of NK cells and T cells, as well as their secretion of cytokines such as IFN-γ and the like. It can avoid the negative feedback of IL-18 pathway activation caused by IL-18BP antagonism, and has controllable activation activity, better molecular stability, and improved in vivo pharmacokinetic characteristics, which are more conducive to fully exerting anti-tumor efficacy in vivo. It significantly inhibits tumor growth in animal model experiments and holds broad prospects for anti-tumor clinical applications.

## Claims

1. An antibody or an antigen-binding fragment thereof that binds to an IL-18 receptor, wherein the antibody or the antigen-binding fragment thereof binds to IL-18R1 or IL-18RAP and comprises a heavy chain variable region; and
wherein, the complementary determining regions of the heavy chain variable region of the antibody or the antigen-binding fragment thereof that binds to IL-18R1 are any one of the following (1) - (5):
(1) the amino acid sequence of the complementary determining region CDR1 of the heavy chain variable region is set forth in SEQ ID NO. 27, or it is a sequence variant having at least 80% homology to the sequence set forth in SEQ ID NO. 27,
the amino acid sequence of the complementary determining region CDR2 of the heavy chain variable region is set forth in SEQ ID NO. 28, or it is a sequence variant having at least 80% homology to the sequence set forth in SEQ ID NO. 28,
the amino acid sequence of the complementary determining region CDR3 of the heavy chain variable region is set forth in SEQ ID NO. 29 or SEQ ID NO. 51, or it is a sequence variant having at least 80% homology to the sequence set forth in SEQ ID NO. 29 or SEQ ID NO. 51;
(2) the amino acid sequence of the complementary determining region CDR1 of the heavy chain variable region is set forth in SEQ ID NO. 30, or it is a sequence variant having at least 80% homology to the sequence set forth in SEQ ID NO. 30,
the amino acid sequence of the complementary determining region CDR2 of the heavy chain variable region is set forth in SEQ ID NO. 31, or it is a sequence variant having at least 80% homology to the sequence set forth in SEQ ID NO. 31,
the amino acid sequence of the complementary determining region CDR3 of the heavy chain variable region is set forth in SEQ ID NO. 32 or SEQ ID NO. 52, or it is a sequence variant having at least 80% homology to the sequence set forth in SEQ ID NO. 32 or SEQ ID NO. 52;
(3) the amino acid sequence of the complementary determining region CDR1 of the heavy chain variable region is set forth in SEQ ID NO. 33, or it is a sequence variant having at least 80% homology to the sequence set forth in SEQ ID NO. 33,
the amino acid sequence of the complementary determining region CDR2 of the heavy chain variable region is set forth in SEQ ID NO. 34 or SEQ ID NO. 53, or it is a sequence variant having at least 80% homology to the sequence set forth in SEQ ID NO. 34 or SEQ ID NO. 53,
the amino acid sequence of the complementary determining region CDR3 of the heavy chain variable region is set forth in SEQ ID NO. 35, or it is a sequence variant having at least 80% homology to the sequence set forth in SEQ ID NO. 35;
(4) the amino acid sequence of the complementary determining region CDR1 of the heavy chain variable region is set forth in SEQ ID NO. 36, or it is a sequence variant having at least 80% homology to the sequence set forth in SEQ ID NO. 36,
the amino acid sequence of the complementary determining region CDR2 of the heavy chain variable region is set forth in SEQ ID NO. 37 or SEQ ID NO. 54, or it is a sequence variant having at least 80% homology to the sequence set forth in SEQ ID NO. 37 or SEQ ID NO. 54,
the amino acid sequence of the complementary determining region CDR3 of the heavy chain variable region is set forth in SEQ ID NO. 38 or SEQ ID NO. 55, or it is a sequence variant having at least 80% homology to the sequence set forth in SEQ ID NO. 38 or SEQ ID NO. 55; and
(5) the amino acid sequence of the complementary determining region CDR1 of the heavy chain variable region is set forth in SEQ ID NO. 39, or it is a sequence variant having at least 80% homology to the sequence set forth in SEQ ID NO. 39,
the amino acid sequence of the complementary determining region CDR2 of the heavy chain variable region is set forth in SEQ ID NO. 40, or it is a sequence variant having at least 80% homology to the sequence set forth in SEQ ID NO. 40,
the amino acid sequence of the complementary determining region CDR3 of the heavy chain variable region is set forth in SEQ ID NO. 41, or it is a sequence variant having at least 80% homology to the sequence set forth in SEQ ID NO. 41; and
the complementary determining regions of the heavy chain variable region of the antibody or the antigen-binding fragment thereof that binds to IL-18RAP is any one of the following (6) - (8):
(6) the amino acid sequence of the complementary determining region CDR1 of the heavy chain variable region is set forth in SEQ ID NO. 42, or it is a sequence variant having at least 80% homology to the sequence set forth in SEQ ID NO. 42,
the amino acid sequence of the complementary determining region CDR2 of the heavy chain variable region is set forth in SEQ ID NO. 43 or SEQ ID NO. 56, or it is a sequence variant having at least 80% homology to the sequence set forth in SEQ ID NO. 43 or SEQ ID NO. 56,
the amino acid sequence of the complementary determining region CDR3 of the heavy chain variable region is set forth in SEQ ID NO. 44, or it is a sequence variant having at least 80% homology to the sequence set forth in SEQ ID NO. 44;
(7) the amino acid sequence of the complementary determining region CDR1 of the heavy chain variable region is set forth in SEQ ID NO. 45, or it is a sequence variant having at least 80% homology to the sequence set forth in SEQ ID NO. 45,
the amino acid sequence of the complementary determining region CDR2 of the heavy chain variable region is set forth in SEQ ID NO. 46 or SEQ ID NO. 57, or it is a sequence variant having at least 80% homology to the sequence set forth in SEQ ID NO. 46 or SEQ ID NO. 57,
the amino acid sequence of the complementary determining region CDR3 of the heavy chain variable region is set forth in SEQ ID NO. 47, or it is a sequence variant having at least 80% homology to the sequence set forth in SEQ ID NO. 47; and
(8) the amino acid sequence of the complementary determining region CDR1 of the heavy chain variable region is set forth in SEQ ID NO. 48, or it is a sequence variant having at least 80% homology to the sequence set forth in SEQ ID NO. 48,
the amino acid sequence of the complementary determining region CDR2 of the heavy chain variable region is set forth in SEQ ID NO. 49 or SEQ ID NO. 58, or it is a sequence variant having at least 80% homology to the sequence set forth in SEQ ID NO. 49 or SEQ ID NO. 58,
the amino acid sequence of the complementary determining region CDR3 of the heavy chain variable region is set forth in SEQ ID NO. 50, or it is a sequence variant having at least 80% homology to the sequence set forth in SEQ ID NO. 50.

2. The antibody or the antigen-binding fragment thereof that binds to the IL-18 receptor of claim 1, wherein the sequence variant having at least 80% homology is a sequence obtained by substituting any one amino acid of an original sequence with histidine; and the original sequence is any one sequence set forth in SEQ ID NOs. 27-58; and
preferably, a total of only one amino acid in the amino acid sequences of CDR1, CDR2, and CDR3 of each antibody or the antigen-binding fragment thereof is substituted with histidine.

3. The antibody or the antigen-binding fragment thereof that binds to the IL-18 receptor of claim 1 or 2, wherein the amino acid sequence of the heavy chain variable region of the antibody or the antigen-binding fragment thereof that binds to IL-18R1 is set forth in any one of SEQ ID NOs. 7-11, or set forth in any one of SEQ ID NOs. 20-23, or it is a sequence variant having at least 80% homology to any one of SEQ ID NOs. 7-11 and SEQ ID NOs. 20-23; and/or,
the amino acid sequence of the heavy chain variable region of the antibody or the antigen-binding fragment thereof that binds to IL-18RAP is set forth in any one of SEQ ID NOs. 12-14, or set forth in any one of SEQ ID NOs. 24-26, or it is a sequence variant having at least 80% homology to any one of SEQ ID NOs. 12-14 and SEQ ID NOs. 24-26; and
preferably, the sequence variant having at least 80% homology is a sequence obtained by substituting a total of one amino acid in CDR1, CDR2, and CDR3 of any sequence set forth in SEQ ID NOs. 7-14 and SEQ ID NOs. 20-26 with histidine.

4. The antibody or the antigen-binding fragment thereof that binds to the IL-18 receptor of claim 3, wherein for the sequence set forth in SEQ ID NO. 7 or SEQ ID NO. 20, the sequence variant having at least 80% homology is a sequence obtained by substituting the amino acid at position 27, 28, 29, 30, 31, 32, 34, 35, 50, 51, 53, 54, 55, 56, 57, 58, 59, 60, 61, 62, 63, 64, 65, 98, 99, 100, 102, 103, 104, 105, 106, 107, 108, 109 or 110 of the sequence set forth in SEQ ID NO. 7 or SEQ ID NO. 20 with histidine; and/or,
for the sequence set forth in SEQ ID NO. 10 or SEQ ID NO. 23, the sequence variant having at least 80% homology is a sequence obtained by substituting the amino acid at position 31, 32, 33, 48, 49, 50, 51, 52, 53, 54, 55, 56, 57, 58, 59, 60, 61, 62, 63, 96, 97, 98, 99, 100, 101, 102, 103, 104, 105, 107, 108, 109, 110, 111, 112 or 29 of the sequence set forth in SEQ ID NO. 10 or SEQ ID NO. 23 with histidine, or a sequence obtained by substituting the amino acids at positions 52 and 59, 52 and 105, 52 and 107, 59 and 99, 59 and 105, 59 and 107, 59 and 109, 99 and 107, 105 and 109, 52 and 112, 59 and 112, 101 and 112, or 105 and 112 of the sequence set forth in SEQ ID NO. 10 or SEQ ID NO. 23 with histidine; and/or,
for the sequence set forth in SEQ ID NO. 13 or SEQ ID NO. 25, the sequence variant having at least 80% homology is a sequence obtained by substituting the amino acid at position 161, 162, 163, 164, 179, 180, 181, 182, 183, 184, 185, 186, 187, 188, 189, 190, 191, 192, 193, 194, 195, 228, 229, 230, 231, 232, 233, 234 or 235 of the sequence set forth in SEQ ID NO. 13 or SEQ ID NO. 25 with histidine.

5. The antibody or the antigen-binding fragment thereof that binds to the IL-18 receptor of any of claims 1 to 4, wherein the antibody or the antigen-binding fragment thereof is a single-domain antibody, a minibody, a bispecific antibody, a multispecific antibody, a monoclonal antibody, a single-chain antibody, Fab, Fab', F(ab')₂, Fd, Fv, scFv, BsFv, dsFv or (dsFv)₂; and/or,
the antibody or the antigen-binding fragment thereof is a camelid-derived antibody, a humanized antibody, a murine-derived antibody, a rabbit-derived antibody, or a chimeric antibody.

6. A bispecific antibody comprising the antibody or the antigen-binding fragment thereof that binds to the IL-18 receptor of any one of claims 1 to 5;
preferably, the bispecific antibody comprises one or more antibodies or antigen-binding fragments thereof that bind to IL-18R1 and one or more antibodies or antigen-binding fragments thereof that bind to IL-18RAP.

7. The bispecific antibody of claim 6, wherein in the bispecific antibody, the amino acid sequence of the antibody or the antigen-binding fragment thereof that binds to IL-18R1 is set forth in SEQ ID NO. 7, and the amino acid sequence of the antibody or the antigen-binding fragment thereof that binds to IL-18RAP is set forth in SEQ ID NO. 12; or
the amino acid sequence of the antibody or the antigen-binding fragment thereof that binds to IL-18R1 is set forth in SEQ ID NO. 8, and the amino acid sequence of the antibody or the antigen-binding fragment thereof that binds to IL-18RAP is set forth in SEQ ID NO. 14; or
the amino acid sequence of the antibody or the antigen-binding fragment thereof that binds to IL-18R1 is set forth in SEQ ID NO. 8, and the amino acid sequence of the antibody or the antigen-binding fragment thereof that binds to IL-18RAP is set forth in SEQ ID NO. 13; or
the amino acid sequence of the antibody or the antigen-binding fragment thereof that binds to IL-18R1 is set forth in SEQ ID NO. 9, and the amino acid sequence of the antibody or the antigen-binding fragment thereof that binds to IL-18RAP is set forth in SEQ ID NO. 14; or
the amino acid sequence of the antibody or the antigen-binding fragment thereof that binds to IL-18R1 is set forth in SEQ ID NO. 9, and the amino acid sequence of the antibody or the antigen-binding fragment thereof that binds to IL-18RAP is set forth in SEQ ID NO. 13; or
the amino acid sequence of the antibody or the antigen-binding fragment thereof that binds to IL-18R1 is set forth in SEQ ID NO. 10, and the amino acid sequence of the antibody or the antigen-binding fragment thereof that binds to IL-18RAP is set forth in SEQ ID NO. 13; or
the amino acid sequence of the antibody or the antigen-binding fragment thereof that binds to IL-18R1 is set forth in SEQ ID NO. 11, and the amino acid sequence of the antibody or the antigen-binding fragment thereof that binds to IL-18RAP is set forth in SEQ ID NO. 12; or
the amino acid sequence of the antibody or the antigen-binding fragment thereof that binds to IL-18R1 is set forth in SEQ ID NO. 20, and the amino acid sequence of the antibody or the antigen-binding fragment thereof that binds to IL-18RAP is set forth in SEQ ID NO. 24; or
the amino acid sequence of the antibody or the antigen-binding fragment thereof that binds to IL-18R1 is set forth in SEQ ID NO. 21, and the amino acid sequence of the antibody or the antigen-binding fragment thereof that binds to IL-18RAP is set forth in SEQ ID NO. 26; or
the amino acid sequence of the antibody or the antigen-binding fragment thereof that binds to IL-18R1 is set forth in SEQ ID NO. 21, and the amino acid sequence of the antibody or the antigen-binding fragment thereof that binds to IL-18RAP is set forth in SEQ ID NO. 25; or
the amino acid sequence of the antibody or the antigen-binding fragment thereof that binds to IL-18R1 is set forth in SEQ ID NO. 22, and the amino acid sequence of the antibody or the antigen-binding fragment thereof that binds to IL-18RAP is set forth in SEQ ID NO. 26; or
the amino acid sequence of the antibody or the antigen-binding fragment thereof that binds to IL-18R1 is set forth in SEQ ID NO. 22, and the amino acid sequence of the antibody or the antigen-binding fragment thereof that binds to IL-18RAP is set forth in SEQ ID NO. 25; or
the amino acid sequence of the antibody or the antigen-binding fragment thereof that binds to IL-18R1 is set forth in SEQ ID NO. 23, and the amino acid sequence of the antibody or the antigen-binding fragment thereof that binds to IL-18RAP is set forth in SEQ ID NO. 25; or
the amino acid sequence of the antibody or the antigen-binding fragment thereof that binds to IL-18R1 is set forth in SEQ ID NO. 11, and the amino acid sequence of the antibody or the antigen-binding fragment thereof that binds to IL-18RAP is set forth in SEQ ID NO. 24; or
the amino acid sequence of the antibody or the antigen-binding fragment thereof that binds to IL-18R1 is a sequence obtained by substituting the amino acid at position 27, 28, 29, 30, 31, 32, 34, 35, 50, 51, 53, 54, 55, 56, 57, 58, 59, 60, 61, 62, 63, 64, 65, 98, 99, 100, 102, 103, 104, 105, 106, 107, 108, 109 or 110 of the sequence set forth in SEQ ID NO. 20 with histidine, and the amino acid sequence of the antibody or the antigen-binding fragment thereof that binds to IL-18RAP is set forth in SEQ ID NO. 24; or
the amino acid sequence of the antibody or the antigen-binding fragment thereof that binds to IL-18R1 is a sequence obtained by substituting the amino acid at position 31, 32, 33, 48, 49, 50, 51, 52, 53, 54, 55, 56, 57, 58, 59, 60, 61, 62, 63, 96, 97, 98, 99, 100, 101, 102, 103, 104, 105, 107, 108, 109, 110, 111, 112 or 29 of the sequence set forth in SEQ ID NO. 23 with histidine, or by substituting the amino acids at positions 52 and 59, 52 and 105, 52 and 107, 59 and 99, 59 and 105, 59 and 107, 59 and 109, 99 and 107, 105 and 109, 52 and 112, 59 and 112, 101 and 112, or 105 and 112 of the sequence set forth in SEQ ID NO. 23 with histidine; the amino acid sequence of the antibody or the antigen-binding fragment thereof that binds to IL-18RAP is set forth in SEQ ID NO. 25; or
the amino acid sequence of the antibody or the antigen-binding fragment thereof that binds to IL-18R1 is set forth in SEQ ID NO. 23, the amino acid sequence of the antibody or the antigen-binding fragment thereof that binds to IL-18RAP is a sequence obtained by substituting the amino acid at position 161, 162, 163, 164, 179, 180, 181, 182, 183, 184, 185, 186, 187, 188, 189, 190, 191, 192, 193, 194, 195, 228, 229, 230, 231, 232, 233, 234 or 235 of the sequence set forth in SEQ ID NO. 25 with histidine.

8. The bispecific antibody of claim 7 comprising any of the following domains (1) - (21):
(1) the amino acid sequence arranged sequentially from the N-terminus to the C-terminus as follows: the sequence set forth in SEQ ID NO. 7, a linker, and the sequence set forth in SEQ ID NO. 12;
(2) the amino acid sequence arranged sequentially from the N-terminus to the C-terminus as follows: the sequence set forth in SEQ ID NO. 8, a linker, and the sequence set forth in SEQ ID NO. 14;
(3) the amino acid sequence arranged sequentially from the N-terminus to the C-terminus as follows: the sequence set forth in SEQ ID NO. 8, a linker, and the sequence set forth in SEQ ID NO. 13;
(4) the amino acid sequence arranged sequentially from the N-terminus to the C-terminus as follows: the sequence set forth in SEQ ID NO. 9, a linker, and the sequence set forth in SEQ ID NO. 14;
(5) the amino acid sequence arranged sequentially from the N-terminus to the C-terminus as follows: the sequence set forth in SEQ ID NO. 9, a linker, and the sequence set forth in SEQ ID NO. 13;
(6) the amino acid sequence arranged sequentially from the N-terminus to the C-terminus as follows: the sequence set forth in SEQ ID NO. 10, a linker, and the sequence set forth in SEQ ID NO. 13;
(7) the amino acid sequence arranged sequentially from the N-terminus to the C-terminus as follows: the sequence set forth in SEQ ID NO. 12, a linker, and the sequence set forth in SEQ ID NO. 7;
(8) the amino acid sequence arranged sequentially from the N-terminus to the C-terminus as follows: the sequence set forth in SEQ ID NO. 13, a linker, and the sequence set forth in SEQ ID NO. 9;
(9) the amino acid sequence arranged sequentially from the N-terminus to the C-terminus as follows: the sequence set forth in SEQ ID NO. 13, a linker, and the sequence set forth in SEQ ID NO. 10;
(10) the amino acid sequence arranged sequentially from the N-terminus to the C-terminus as follows: the sequence set forth in SEQ ID NO. 12, a linker, and the sequence set forth in SEQ ID NO. 11;
(11) the amino acid sequence arranged sequentially from the N-terminus to the C-terminus as follows: the sequence set forth in SEQ ID NO. 20, a linker, and the sequence set forth in SEQ ID NO. 24;
(12) the amino acid sequence arranged sequentially from the N-terminus to the C-terminus as follows: the sequence set forth in SEQ ID NO. 21, a linker, and the sequence set forth in SEQ ID NO. 26;
(13) the amino acid sequence arranged sequentially from the N-terminus to the C-terminus as follows: the sequence set forth in SEQ ID NO. 21, a linker, and the sequence set forth in SEQ ID NO.25;
(14) the amino acid sequence arranged sequentially from the N-terminus to the C-terminus as follows: the sequence set forth in SEQ ID NO. 22, a linker, and the sequence set forth in SEQ ID NO. 26;
(15) the amino acid sequence arranged sequentially from the N-terminus to the C-terminus as follows: the sequence set forth in SEQ ID NO. 22, a linker, and the sequence set forth in SEQ ID NO. 25;
(16) the amino acid sequence arranged sequentially from the N-terminus to the C-terminus as follows: the sequence set forth in SEQ ID NO. 23, a linker, and the sequence set forth in SEQ ID NO. 25;
(17) the amino acid sequence arranged sequentially from the N-terminus to the C-terminus as follows: the sequence set forth in SEQ ID NO. 24, a linker, and the sequence set forth in SEQ ID NO. 20;
(18) the amino acid sequence arranged sequentially from the N-terminus to the C-terminus as follows: the sequence set forth in SEQ ID NO. 25, a linker, and the sequence set forth in SEQ ID NO. 22;
(19) the amino acid sequence arranged sequentially from the N-terminus to the C-terminus as follows: the sequence set forth in SEQ ID NO. 25, a linker, and the sequence set forth in SEQ ID NO. 23;
(20) the amino acid sequence arranged sequentially from the N-terminus to the C-terminus as follows: the sequence set forth in SEQ ID NO. 24, a linker, and the sequence set forth in SEQ ID NO. 11;
(21) the amino acid sequence obtained by substituting the amino acid at position 27, 28, 29, 30, 31, 32, 34, 35, 50, 51, 53, 54, 55, 56, 57, 58, 59, 60, 61, 62, 63, 64, 65, 98, 99, 100, 102, 103, 104, 105, 106, 107, 108, 109 or 110 of the sequence set forth in SEQ ID NO. 20 with histidine on the basis of the amino acid arranged sequentially from the N-terminus to the C-terminus as follows: the sequence set forth in; SEQ ID NO. 20, a linker, and the sequence set forth in SEQ ID NO. 24;
(22) the amino acid sequence arranged sequentially from the N-terminus to the C-terminus as follows: a sequence obtained by substituting the amino acid at position 31, 32, 33, 48, 49, 50, 51, 52, 53, 54, 55, 56, 57, 58, 59, 60, 61, 62, 63, 96, 97, 98, 99, 100, 101, 102, 103, 104, 105, 107, 108, 109, 110, 111, 112 or 29 of the sequence set forth in SEQ ID NO. 23 with histidine, or obtained by substituting the amino acids at positions 52 and 59, 52 and 105, 52 and 107, 59 and 99, 59 and 105, 59 and 107, 59 and 109, 99 and 107, 105 and 109, 52 and 112, 59 and 112, 101 and 112, or 105 and 112 of the sequence set forth in SEQ ID NO. 23 with histidine, a linker, and the sequence set forth in SEQ ID NO. 25; and
(23) the amino acid sequence arranged sequentially from the N-terminus to the C-terminus as follows: the sequence set forth in SEQ ID NO. 23, a linker, and a sequence obtained by substituting the amino acid at position 161, 162, 163, 164, 179, 180, 181, 182, 183, 184, 185, 186, 187, 188, 189, 190, 191, 192, 193, 194, 195, 228, 229, 230, 231, 232, 233, 234 or 235 of the sequence set forth in SEQ ID NO. 25.

9. The bispecific antibody of any one of claims 6 to 8, wherein the bispecific antibody comprises any one or more selected from KappaL, CH1, CH2, CH3, a hinge region, and KIH structure.

10. A fusion protein comprising the antibody or the antigen-binding fragment thereof that binds to the IL-18 receptor of any one of claims 1 to 5, or the bispecific antibody of any one of claims 6 to 9;
preferably, the fusion protein is obtained by fusing the antibody or the antigen-binding fragment thereof that binds to the IL-18 receptor of any one of claims 1 to 5, or the bispecific antibody of any one of claims 6 to 9, with other proteins; and
more preferably, said other proteins comprise one or more selected from a protein tag, Fab, Fc, HSA, a cytokine or an antibody.

11. A bifunctional protein comprising the antibody or the antigen-binding fragment thereof that binds to the IL-18 receptor of any one of claims 1 to 5, or the bispecific antibody of any one of claims 6 to 9, and further comprising a targeting protein, an antibody, or a cytokine;
preferably, the antibody is an immune checkpoint antibody and/or a tumor-targeted antibody.

12. A nucleic acid molecule encoding the antibody or the antigen-binding fragment thereof that binds to the IL-18 receptor of any one of claims 1 to 5, the bispecific antibody of any one of claims 6 to 9, the fusion protein of claim 10, or the bifunctional protein of claim 11.

13. A biological material comprising an expression cassette, a vector or a host cell containing the nucleic acid molecule of claim 12.

14. A method for preparing the antibody or the antigen-binding fragment thereof that binds to the IL-18 receptor of any one of claims 1 to 5, the bispecific antibody of any one of claims 6 to 9, the fusion protein of claim 10, or the bifunctional protein of claim 11, comprising: introducing a nucleic acid molecule encoding the antibody or the antigen-binding fragment thereof that binds to the IL-18 receptor of any one of claims 1 to 5, the bispecific antibody of any one of claims 6 to 9, the fusion protein of claim 10, or the bifunctional protein of claim 11, into a host cell to obtain a recombinant host cell; cultivating the recombinant host cell, and isolating and obtaining the antibody or the antigen-binding fragment thereof that binds to the IL-18 receptor, or the bispecific antibody or the fusion protein.

15. Any of the following uses of the antibody or the antigen-binding fragment thereof that binds to the IL-18 receptor of any one of claims 1 to 5, the bispecific antibody of any one of claims 6 to 9, the fusion protein of claim 10, the bifunctional protein of claim 11, the nucleic acid molecule of claim 12, or the biological material of claim 13:
(1) use in the preparation of anti-tumor drugs;
(2) use in the preparation of drugs that enhance a body's immune response;
(3) use in the preparation of drugs for activating an IL-18 signaling pathway;
(4) use in the preparation of drugs for stimulating the secretion of INF γ and/or GZMB;
(5) use in the preparation of IL-18 receptor activators;
(6) use in the preparation of drugs for the prevention or treatment of IL-18 receptor related diseases; and
(7) use in the preparation of reagents for detecting IL-18 receptor content.

16. An IL-18 receptor activator comprising the antibody or the antigen-binding fragment thereof that binds to the IL-18 receptor of any one of claims 1 to 5, the bispecific antibody of any one of claims 6 to 9, the fusion protein of claim 10, or the bifunctional protein of claim 11.

17. A pharmaceutical composition comprising the antibody or the antigen-binding fragment thereof that binds to the IL-18 receptor of any one of claims 1 to 5, the bispecific antibody of any one of claims 6 to 9, the fusion protein of claim 10, or the bifunctional protein of claim 11.

18. A detection reagent comprising the antibody or the antigen-binding fragment thereof that binds to the IL-18 receptor of any one of claims 1 to 5, the bispecific antibody of any one of claims 6 to 9, the fusion protein of claim 10, or the bifunctional protein of claim 11.
